(19) Europäisches Patentamt / European Patent Office / Office européen des brevets

(11) **EP 4 342 913 B1**

(12) **EUROPEAN PATENT SPECIFICATION**

(45) Date of publication and mention
of the grant of the patent:
**17.12.2025 Bulletin 2025/51**

(21) Application number: **22804030.9**

(22) Date of filing: **19.05.2022**

(51) International Patent Classification (IPC):
*A61K 40/11* (2025.01)   *A61K 40/31* (2025.01)
*A61K 40/42* (2025.01)   *A61P 35/00* (2006.01)
*A61P 37/02* (2006.01)   *A61P 37/06* (2006.01)
*C07K 14/725* (2006.01)   *C07K 16/28* (2006.01)

(52) Cooperative Patent Classification (CPC):
**A61P 35/00; A61K 40/11; A61K 40/31;
A61K 40/4215; A61P 37/02; A61P 37/06;
C07K 14/7051; C07K 16/2878;** A61K 2239/31;
A61K 2239/38; C07K 2317/622; C07K 2319/03

(86) International application number:
**PCT/CN2022/093782**

(87) International publication number:
**WO 2022/242710 (24.11.2022 Gazette 2022/47)**

(54) **CHIMERIC ANTIGEN RECEPTOR MOLECULE FOR SPECIFICALLY RECOGNIZING BAFF-R AND APPLICATION OF CHIMERIC ANTIGEN RECEPTOR MOLECULE**

CHIMÄRES ANTIGENREZEPTORMOLEKÜL ZUR SPEZIFISCHEN ERKENNUNG VON BAFF-R UND ANWENDUNG EINES CHIMÄREN ANTIGENREZEPTORMOLEKÜLS

MOLÉCULE DE RÉCEPTEUR ANTIGÉNIQUE CHIMÉRIQUE POUR RECONNAÎTRE DE MANIÈRE SPÉCIFIQUE BAFF-R ET UTILISATION D'UNE MOLÉCULE DE RÉCEPTEUR ANTIGÉNIQUE CHIMÉRIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.05.2021 PCT/CN2021/094605**

(43) Date of publication of application:
**27.03.2024 Bulletin 2024/13**

(73) Proprietors:
 • **Shanghai Escugen Biotechnology Co., Ltd.
  Shanghai 201203 (CN)**
 • **Guangzhou Yinnuolai Biotechnology Co., Ltd.
  Guangzhou, Guangdong 510653 (CN)**

(72) Inventors:
 • **ZHANG, Xinmin
  Shanghai 201203 (CN)**
 • **NIAN, Weihong
  Shanghai 201203 (CN)**
 • **XU, Chuanying
  Shanghai 201203 (CN)**
 • **HE, Feng
  Shanghai 201203 (CN)**
 • **ZHOU, Qing
  Shanghai 201203 (CN)**
 • **ZHANG, Jianbing
  Guangzhou, Guangdong 510100 (CN)**

(74) Representative: **Abel & Imray LLP
  Westpoint Building
  James Street West
  Bath BA1 2DA (GB)**

(56) References cited:
  WO-A1-2017/214167    WO-A1-2021/163989
  WO-A1-2021/168169    CN-A- 101 851 291
  CN-A- 112 292 137    US-A1- 2019 201 442

EP 4 342 913 B1

• DONG ZHENYUAN; CHENG WESLEY A.; SMITH D. LYNNE; HUANG BRIAN; ZHANG TIANTIAN; CHANG WEN-CHUNG; WANG XIULI; FORMAN STEPHEN J.; KWAK : "Antitumor efficacy of BAFF-R targeting CAR T cells manufactured under clinic-ready conditions", CANCER IMMUNOLOGY IMMUNOTHERAPY, SPRINGER, BERLIN/HEIDELBERG, vol. 69, no. 10, 25 May 2020 (2020-05-25), Berlin/Heidelberg , pages 2139 - 2145, XP037253279, ISSN: 0340-7004, DOI: 10.1007/s00262-020-02614-8
• L M QIN, QIN HONG, DONG ZHENYUAN, WANG XIULI, CHENG WESLEY A, WEN FENG, XUE WEILI, SUN HAN, WALTER MIRIAM, WEI GUOWEI, SMITH D LYN: "CAR T cells targeting BAFF-R can overcome CD19 antigen loss in B cell malignancies", SCI. TRANSL. MED, 25 September 2019 (2019-09-25), XP055706529, Retrieved from the Internet <URL:https://stm. sciencemag.org/content/11/511/eaaw9414.full. pdf> [retrieved on 20200618], DOI: 10.1126/ scitranslmed.aaw9414

Remarks:
    The complete document including Reference Table(s) and the Sequence Listing(s) can be downloaded from the EPO website

**Description**

## TECHNICAL FIELD

The present application relates to an engineered specific antigen recognition receptor molecule and an engineered immune effector cell comprising the receptor molecule.

## BACKGROUND OF THE ART

In recent decades, traditional tumor treatment methods (surgery, chemotherapy, radiotherapy, etc.) have made great progress, but there are still huge challenges in terms of survival rate, recurrence and metastasis. With the rapid development of tumor biology and immunology research, immunotherapy has gradually become a hot spot in tumor treatment in recent years. Among them, Chimeric Antigen Receptor T-cell(CAR-T) therapy has become one of the most promising tumor immunotherapies at present.

T lymphocytes play a major role in the tumor immune response and have a strong ability to kill tumor cells, but their function is limited by MHC. Using genetic engineering methods, the single-chain antibody that recognizes the target antigen in a non-MHC-restricted manner is linked with the hinge region, transmembrane domain, and T cell activation motif to assemble a CAR structure, and the CAR structure is integrated into the T cell with the help of a virus and other vectors, and finally enable T cells to specifically bind to antigens associated with the surface of tumor cells and be activated, that is, CAR-T. On the one hand, CAR-T directly kills tumor cells by releasing perforin and granzyme B; on the other hand, activated CAR-T further releases cytokines to recruit human endogenous immune cells to kill tumor cells, achieving the purpose of effectively inhibiting tumors. At the same time, immune memory T cells can also be formed to obtain a specific long-term anti-tumor mechanism.

In general, a chimeric antigen receptor consists of an extracellular domain, a transmembrane domain, and an activation stimulation domain. Usually, the extracellular domain is composed of an antigen recognition domain and a connecting hinge region. The antigen recognition domain is the basis for CAR to specifically bind tumor antigens. Its main structure is an scFv, which is usually composed of a light chain(VL) and a heavy chain(VH) of a monoclonal antibody linked by a polypeptide, retaining the specificity of the antibody to the antigen. The hinge region connects the scFv and the transmembrane domain, and the hinge region of most CARs is derived from the hinge of IgG or the extracellular region of CD8$\alpha$/CD28. The transmembrane domain connects the extracellular domain of the CAR with the intracellular activation stimulation domain and anchors the receptor to the T cell membrane. Commonly used transmembrane domains include those membrane domains derived from CD4, CD8$\alpha$, CD28 and CD3$\zeta$. The activation stimulation domain consists of an intracellular costimulatory domain and a signaling domain, and the costimulatory domain is usually from the CD28 receptor family(CD28, ICOS) or tumor necrosis factor receptor family(4-1BB, OX40, CD27), and the signaling domain is usually the T cell receptor TCR/CD3$\zeta$ chain, containing an immunoreceptor tyrosine-based activation motif(ITAM). The activation stimulation domain plays a key role in the process of mediating T cell signal transmission until T cell activation and proliferation, and finally completes the killing of tumors.

B-cell malignancies are high-incidence malignancies, and there are about 300,000 new patients worldwide each year, with an annual growth rate of 5%-7%. Chimeric antigen receptor T cells(CAR-T) have been widely used in the treatment of B-cell malignancies, and CAR-T cell therapy targeting CD19 has a good effect on refractory B-cell malignancies. Response rates in patients with acute lymphoblastic leukemia(ALL) and various non-Hodgkin's lymphoma subtypes are as high as 80%-90%. However, only about 40% to 50% of patients have long-term efficacy, and CD19 downregulation/negative relapse occurs in about 30% of patients. No matter it is the down-regulated expression or even complete loss of CD19 caused by genetic modification or the transformation of CD19 into other forms caused by cutting that results in the inability for CD19CAR-T cells to recognize, it brings great challenges to CD19 CAR-T therapy. Although the clinical prognosis of B cell malignancies has been greatly improved with the breakthrough of immunotherapy and targeted drugs in recent years, the high recurrence rate is still a problem. Therefore, exploring other antigenic targets highly expressed on the surface of B cells is an important research direction to solve relapsed and refractory B cell malignancies.

B lymphocyte stimulator(BLyS for short), a member of the tumor necrosis factor(TNF) superfamily, also known as TNF-and ApoL related leukocyte-expressed ligand 1(TALL-1), TNF homologue that activates apoptosis, nuclear factor-$\kappa$B and c-Jun NH2-terminal kinase(THANK), TNF superfamily member 13B(TNFSF13B), B cell-activating factor(BAFF) and zTNF4, belongs to type II transmembrane proteins, can specifically bind to B cells and plays a key role in B cell survival, proliferation, development and differentiation. BAFF has three receptors, namely BAFF receptor(BAFF-R), B cell maturation antigen(BCMA) and TNFR homology transmembrane activator and calcium modulator and cyclophilin ligand intergrator(TACI), all of which are type III transmembrane proteins. BCMA and TACI can not only bind to BAFF, but also bind to another member of the TNF ligand family, the proliferation-inducing ligand(APRIL), and BAFF-R is a specific receptor for BAFF and plays a more important role than the other two receptors in the regulatory B lymphocytes.

BAFF-R(B-cell activating factor receptor), also known as tumor necrosis factor superfamily member 13C(TNFRSF13C), is a member of the TNF receptor(TNFR) superfamily on the B cell membrane, and is a type III transmembrane protein lacking signal peptides. The published human BAFF-R amino acid sequence has a total length of 184 amino acids, of which amino acids 1-78 are the extracellular domain, amino acids 79-99 are the transmembrane domain, and amino acids 100-184 are the intracellular region. Its coding gene is located on chromosome 22q13.1. BAFF-R is mainly expressed in B cells, promoting the survival and proliferation of B cells by activating the NF-κB pathway, and is highly expressed on the surface of various B cell malignant tumor cells.

The imbalance of the BAFF-BAFF-R signaling pathway can cause the immune imbalance of the body, including a series of diseases caused by abnormally high expression of BAFF-R in abnormal or normal cells, such as autoimmune diseases, graft-versus-host diseases and tumors. This target covers a wide range of stages in the maturation of B cells, and can target all B cell malignancies except plasma cell lesions(multiple myeloma), having a wide range of applications. In addition, this target is not expressed in stem cells and progenitor B cells, and therefore does not permanently harm the recovery and function reconstitution of normal B cells, having a favorable safety profile.

With the breakthrough of immunotherapy and targeted drugs, the clinical prognosis of B cell malignancies has been greatly improved, but the high recurrence rate is still a problem. Therefore, it is necessary to explore other antigen targets highly expressed on the surface of B cells and therapeutic drugs in order to solve recurrence and the refractory B cell malignancies.

WO 2017/214167 A1 relates to BAFF-R targeted chimeric antigen receptor (CAR)-modified T-cells and their uses. It discloses T cells expressing a chimeric antigen receptor (CAR) targeted to BAFF-R, which includes a domain that binds BAFF-R, and methods of making and using the same BAFF-R CAR.

## SUMMARY OF THE INVENTION

For diseases caused by the imbalance of the B cell BAFF-BAFF-R signaling pathway, such as B cell malignancies, this application provides a chimeric antigen receptor molecule(CAR molecule) specifically recognizing BAFF-R and an engineered immune effector cell(e.g., chimeric antigen receptor T cells(CAR-T)). Studies have proved that the chimeric antigen receptor T cells targeting BAFF-R of the present application have a strong killing effect on tumor cells and an *in vivo* tumor suppressing effect, and have potential for development.

The present invention provides a chimeric antigen receptor molecule specifically recognizing BAFF-R, comprising a specific recognition domain and an activation stimulation domain, and a transmembrane domain located between the specific recognition domain and the activation stimulation domain, wherein, the specific recognition domain comprises a heavy chain variable region and a light chain variable region, wherein,

the heavy chain variable region comprises three complementary determining regions CDR H1, CDR H2 and CDR H3, and CDR H1 comprises an amino acid sequence set forth in SEQ ID NO:6, CDR H2 comprises an amino acid sequence set forth in SEQ ID NO:7, and CDR H3 comprises an amino acid sequence set forth in SEQ ID NO: 8;
the light chain variable region comprises CDR L1, CDR L2 and CDR L3, and the CDR L1 comprises an amino acid sequence set forth in SEQ ID NO:10, CDR L2 comprises an amino acid sequence set forth in SEQ ID NO: 11, and CDR L3 comprises an amino acid sequence set forth in SEQ ID NO: 12.

Also provided are: a nucleic acid molecule encoding the chimeric antigen receptor molecule specifically recognizing BAFF-R, as defined in the appended claims; an engineered immune effector cell comprising the chimeric antigen receptor molecule specifically recognizing BAFF-R, as defined in the appended claims; a pharmaceutical composition comprising the chimeric antigen receptor molecule, nucleic acid molecule, or engineered immune effector cell, as defined in the appended claims, and uses of said pharmaceutical composition.

The present application relates to a chimeric antigen receptor molecule specifically recognizing BAFF-R, which comprises a specific recognition domain and an activation stimulation domain, and a transmembrane domain between the specific recognition domain and the activation stimulation domain, wherein the specific recognition domain comprises a heavy chain variable region and a light chain variable region, wherein the heavy chain variable region comprises three complementarity determining regions CDR H1, CDR H2 and CDR H3, and the CDR H1 comprises an amino acid sequence set forth in SEQ ID NO:6, the CDR H2 comprises an amino acid sequence set forth in SEQ ID NO:7, and the CDR H3 comprises an amino acid sequence set forth in SEQ ID NO:8, and the light chain variable region comprises CDR L1, CDR L2 and CDR L3, and the CDR L1 comprises an amino acid sequence set forth in SEQ ID NO: 10, the CDR L2 comprises an amino acid sequence set forth in SEQ ID NO:11, and the CDR L3 comprises an amino acid sequence set forth in SEQ ID NO:12. The CDR H1, CDR H2, CDR H3 and CDR L1, CDR L2, CDR L3 are determined according to Kabat et al.(Kabat et al., Sequences of Proteins of Immunological Interest, Fifth Edition, NIH Publication 91-3242, Bethesda MD(1991) , vols.1-3).

The heavy chain variable region and the light chain variable region of the chimeric antigen receptor molecule specifically recognizing BAFF-R may further comprise a human antibody framework region. In some embodiments, the human antibody framework region is a human common framework region. In some embodiments, the human common framework region comprises the framework region sequence of the kappa I subgroup(xI subgroup) of VL and the framework region sequence of the III subgroup(VH III subgroup) of VH. The framework region sequence is determined according to Kabat et al.(Kabat et al., Sequences of Proteins of Immunological Interest, Fifth Edition, NIH Publication 91-3242, Bethesda MD(1991), vols. 1-3). In some embodiments, the human common framework region comprises 1 to 15, 1 to 10, 2 to 9, 3 to 8, 4 to 7 or 5 to 6 amino acid changes.

The chimeric antigen receptor molecule specifically recognizing BAFF-R provided in this application can specifically recognize and bind to BAFF-R and activate downstream signaling pathways to cause, promote or enhance the immune response with regards to BAFF-R, for example, specifically kill target cells expressing BAFF-R in vitro or in vivo, and finally achieve the purpose of treating or preventing diseases caused by the imbalance of the B cell BAFF-BAFF-R signaling pathway.

## BRIEF DESCRIPTION OF THE DRAWINGS

FIG. 1 shows an example of the structure of the chimeric antigen receptor for BAFF-R of the present invention.
FIG. 2 shows an exemplary plasmid map of an anti-BAFF-R chimeric antigen receptor lentiviral expression vector pCDH-EFla-H90-11CAR-4-1BB-EGFRt.
FIG. 3 shows a plasmid map of the lentiviral expression vector pCDH-EF1a-EGFRt-AT-Free(delete T2A) as a control.
FIG. 4 shows the result of flow cytometry detection of CAR positive rate using FITC-EGFRt antibody.
FIG. 5 shows the results of flow cytometry detection of CAR positive rate using BAFF-R protein.
FIG. 6 shows a graph of the results of specific killing of Nalm6-luciferase target cells by H90-11 CAR-T cells.
FIGs. 7A to 7B show the results of secretion of IL-2(Figure A) and IFN-$\gamma$(Figure B) in the supernatant after H90-11 CAR-T cells were co-cultured with Nalm6-luciferase target cells.
FIGs. 8A to 8C show a graph of anti-tumor effects of H90-11 CAR-T in mice. Among them, FIG. 8A shows a graph of *in vivo* imaging results of mice at different time points; FIG. 8B shows a graph of absolute luminescence in mice at different time points; FIG. 8C shows a graph of body weight changes in mice within 22 days.

## DETAIL DESCRIPTION OF THE INVENTION

In order to further improve and enhance the therapeutic effect on B-cell malignancies, this application designs a new type of chimeric antigen receptor molecule recognizing BAFF-R protein that specifically and highly expressed on the surface of B cells, and provides a nucleic acid molecule encoding the receptor, the engineered immune effector cell comprising the receptors, the pharmaceutical composition, and the use thereof for the purpose of treating or preventing diseases caused by imbalances of the B cell BAFF-BAFF-R signaling pathway, such as B cell malignancies.

Definition

For the purpose of interpreting this specification, the following definitions will apply, and where appropriate, terms used in the singular will also include the plural and vice versa. In the event of any conflict between any definition set forth below and any document referred to herein, the definition set forth below shall control.

As used herein, "receptor" refers to a biomacromolecule capable of binding to hormones, neurotransmitters, drugs, or intracellular signaling molecules and causing changes in cell functions. According to the location of receptors in the cell, receptors can be divided into two categories: cell membrane receptors and intracellular receptors. A receptor itself contains at least two active sites: one is the active site that recognizes and binds to the ligand(referred to as the "specific recognition domain" in this application); and the other is the activation site that responsible for the production of response(referred to as the "activation stimulation domain" herein). The activation-stimulation domain can produce a response only after the receptor binds to the ligand to form a binary complex and is allosteric, thereby initiating a series of biochemical reactions, and finally causing the effector cell where the receptor is located to produce biological effects. As used herein, the "activation stimulation domain" comprises a signaling domain, or comprises one or more signaling domains and one or more costimulatory domains. Herein, unless otherwise specified, a "signaling domain" provides a primary signal for activating lymphocytes, such as activating T cells or NK cells, while a "costimulatory domain" provides a secondary signal for activating lymphocytes. A transmembrane domain is further included between the antigen recognition region and the activation stimulation domain. In some embodiments, the antigen recognition region and the transmembrane domain are linked by a hinge region.

As used herein, "intracellular domain" and "intracellular region" may be used interchangeably, and may refer to a domain of a receptor molecule that is located in the cell and plays a role in signaling after the receptor binds to a ligand.

As used herein, "chimeric antigen receptor(CAR)" is a class of engineered cell surface receptors, generally expressed on immune cells, which mediates the killing of engineered immune cells against specific target tumor cells or other diseased cells. As a receptor, a CAR also comprises a specific recognition domain and an activation stimulation domain. The specific recognition domain of a CAR can specifically recognize an antigen, so it is also called the antigen recognition region. Usually, the antigen recognition region of a CAR is located outside the cell membrane. In some embodiments, the antigen recognition region is a single chain variable fragment(scFv) of an Ig. A "scFv" comprises a heavy chain variable region(VH) and a light chain variable region(VL) of an Ig. In some specific embodiments, the VL and VH regions are linked together by a peptide chain.

The terms "heavy chain"("CH"), "light chain"("CL"), "light chain variable region"("VL"), "heavy chain variable region"("VH"), and "framework region"("FR") refer to the domains in naturally occurring immunoglobulins and the corresponding domains of synthetic(e.g., recombinant) binding proteins(e.g., humanized antibodies). The basic structural unit of naturally occurring immunoglobulins, such as IgG, is a tetramer with two light chains and two heavy chains. The amino-terminal("N") portion of each chain comprises a variable region of about 100 to 110 or more amino acids primarily responsible for antigen recognition. The carboxy-terminal("C" portion) of each chain defines a constant region. A light chain has a single constant domain and a heavy chain typically has three constant domains and a hinge region. Thus, a naturally occurring light chain structure of an IgG molecule is N-VL-CL-C, and the structure of an IgG heavy chain is N-VH-CH1-H-CH2-CH3-C(where H is the hinge region). Among them, CH1, CH2 and CH3 are the components of the constant region of an antibody's heavy chain, the CH3 region is involved in the binding of a receptor on the cell membrane surface, CH2 is involved in the complement activation pathway and is the complement binding site, and CH1 has an Ig allotype genetic marker. The variable region of IgG consists of complementarity determining regions(CDRs) and non-CDR segments called framework regions. Among them, a CDR contains residues that contact an antigen, and a framework region is used to maintain the structure of the variable region and determine the position of the CDR loop. Thus, VL and VH domains have the following structure: N-FR1-CDR1-FR2-CDR2-FR3-CDR3-FR4-C.

In some embodiments, the specific recognition domain is a polypeptide or protein ligand other than scFv.

As used herein, the term "single-chain variable fragment", "single-chain antibody variable fragment" or "scFv" antibody refers to a form of an antibody only comprising the variable regions of a heavy chain(CH) and a light chain(CL) ligated by a linker peptide. An scFv can be expressed as a single chain polypeptide. An scFv retains the specificity of the intact antibody from which it was derived. The light and heavy chains can be in any order, e.g., VH-linker-VL or VL-linker-VH, as long as the specificity of the scFv for the target antigen is preserved. In some special embodiments, linkers can also be omitted.

As used herein, the term "linker" refers to an oligopeptide or polypeptide region of about 1 to 100 amino acids in length that links together any of the domains/regions of the CAR of the invention. Linkers can be composed of flexible residues such as glycine and serine so that adjacent protein domains are free to move relative to each other. Longer linkers can be used when it is desired to ensure that two adjacent domains do not sterically interfere with each other. Alternative linkers are known to those skilled in the art and may be used in combination with alternative embodiments of the invention.

As used herein, the term "antibody" refers to an intact immunoglobulin or a monoclonal or polyclonal antigen-binding fragment having an Fc region(fragment crystallizable region) or the FcRn-binding fragment of an Fc(referred to herein as an "Fc fragment" or "Fc domain"). An antigen-binding fragment can be produced by recombinant DNA techniques or by enzymatic or chemical cleavage of an intact antibody. Antigen binding fragments specifically include Fab, Fab', F(ab')2, Fv, dAb and complementarity determining region(CDR) fragments, single chain antibodies(scFv), single domain antibodies, chimeric antibodies, diabodies and such polypeptides comprising at least a portion of an immunoglobulin sufficient to confer specific antigen binding to the polypeptide. An Fc domain comprises CH2 and CH3 portions of two heavy chains, and can be produced by recombinant DNA techniques or by enzymatic(e.g., papain cleavage) or by chemical cleavage of intact antibodies. The term "antibody fragment" refers to a fragment of a protein that comprises only a portion of an intact antibody, usually including the antigen-binding site of the intact antibody, and thus retains the ability to bind the antigen. Examples of antibody fragments described herein include, but are not limited to:(i) Fab fragments having VL, CL, VH, and CH1 domains;(ii) Fab' fragments, i.e., a Fab fragment having one or more cysteine residues at the C-terminal of the CH1 domain;(iii) Fd fragments having VH and CH1 domains;(iv) Fd' fragments having VH and CH1 domains and one or more cysteine residues at the C-terminal of the CH1 domains;(v) Fv fragments having VL and VH domains of a single antibody arm;(vi) dAb fragments consisting of a VH domain(Ward et al., Nature 341, 544-546(1989));(vii) isolated CDR regions;(viii) F(ab')2 fragments, bivalent fragments comprising two Fab' fragments bridged by disulfide bonds at the hinge region;(ix) single chain antibody molecules(e.g., single chain Fv; scFv)(Bird et al., Science 242:423-426(1988); and Huston et al., PNAS(USA) 85:5879-5883(1988));(x) "diabodies" having two antigen binding sites, which comprise a heavy chain variable domain(VH) linked to a light chain variable domain(VL) in the same polypeptide chain(see, e.g., EP404,097; WO93/11161; and Hollinger et al., Proc. Natl .Acad.Sci.USA, 90:6444-6448(1993));(xi) "linear antibody", which comprises a pair of tandem Fd segments(VH-CH1-VH-CH1), the Fd segments form a pair of antigen-binding regions together with the complementary light chain polypeptides(Zapata et al.,

Protein Eng. 8(10):1057-1062(1995); and US Patent No. 5,641,870).

As used herein, the term "specifically bind" means that an antibody binds to a specific antigen and does not bind to other antigens other than the specific antigen. The specific antigens may be one or more, and in some embodiments, the specific antigens contain the same or similar antigenic epitopes. In some embodiments, the specific binding has a binding affinity for the contact between the antibody and the antigen of at least $10^{-6}$M. In certain embodiments, the antibody binds with an affinity of at least about $10^{-7}$M, preferably $10^{-8}$M, $10^{-9}$M, $10^{-10}$M, $10^{-11}$M, or $10^{-12}$M.

In this application, the terms "polynucleotide", "nucleic acid" or "nucleic acid molecule" are used interchangeably, including but not limited to DNA, RNA, cDNA(complementary DNA), mRNA(messenger RNA), rRNA(ribosomal RNA) , shRNA(small hairpin RNA), snRNA(small nuclear RNA), snoRNA(short nucleolar RNA), miRNA(microRNA), genomic DNA, synthetic DNA, synthetic RNA and/or tRNA.

As used herein, "vector", "cloning vector" and "expression vector" refer to a vector that can introduce a polynucleotide sequence(such as a foreign gene) into a host cell to transform the host and facilitate the expression(such as transcription and translation) of the introduced sequence. The vectors include plasmids, phages, viruses and the like.

As used herein, the "signaling domain" generally comprises an immune-receptor tyrosine-based activation motif(ITAM), the basic composition of which is: YXXL/V. Wherein Y is tyrosine, L/V refers to leucine or valine, and X can be any amino acid. When the receptor binds to the corresponding ligand, the tyrosine in the ITAM linked to it can be phosphorylated under the action of a type of protein tyrosine kinase PTK linked to the cell membrane, thereby recruiting other free protein kinases or adapter proteins in the cell, transmitting activation signals into the cell. In some embodiments, the "signaling domain" is the intracellular signaling domain of TCRζ(CD3ζ) or FcεRIγ.

As used herein, the "costimulatory domain", also known as "costimulatory signal region", is mainly used to provide a costimulatory signal to enhance the ability of immune cells, including, for example, enhancing the proliferation, survival and/or development of memory cells. In some embodiments, the "costimulatory domain" is selected from CD28, 4-1BB(CD137), OX40(CD134) and the like.

As used herein, the "transmembrane domain", also known as "transmembrane region", refers to a thermodynamically stable protein structural region anchored in the cell membrane. Transmembrane domains can be obtained from natural proteins, such as those derived from T cell receptors(TCR). In some embodiments, the transmembrane domain is selected from the group consisting of transmembrane domains of CD4, CD8α, CD28, and CD3ζ.

As used herein, the "hinge region" is a peptide chain connecting the antigen recognition region and the transmembrane domain, and is usually elastic. In some embodiments, the hinge region is derived from the hinge of IgG or the extracellular region of CD8α/CD28. The "hinge" of IgG refers to the region between the CH1 and CH2 functional regions of IgG, which usually comprises a large amount of proline.

As used herein, the "anti-BAFF-R chimeric antigen receptor" is one of the "receptors specifically recognizing BAFF-R". It specifically refers to a chimeric antigen receptor containing an anti-BAFF-R antibody heavy chain variable region and light chain variable region in the antigen recognition region. The chimeric antigen receptor can specifically recognize BAFF-R, and activate the downstream pathway of the cell where the receptor is located through signaling. In some embodiments, the anti-BAFF-R chimeric antigen receptor is located on the surface of immune cells selected from NK cells, macrophages, neutrophils, T cells, etc. After specifically recognizing BAFF-R, it activates the immune effector cell to produce immune effects such as humoral immunity, cellular immunity, and/or cytotoxicity; or activates the immune cells to further proliferate, etc.

As used herein, an "immune effector cell" refers to a cell(such as T cells and natural killer(NK) cells) capable of achieving immune effects and immune responses(such as immune killing effects and immune response effects) against a target antigen or a target cell.

As used herein, a "CAR-T" is a chimeric antigen receptor T cell, which is a T cell expressing a chimeric antigen receptor molecule on the cell surface, and can recognize a target antigen on a cell surface. A CAR-T has been developed to the fourth generation. Among them, a CAR-T of the first-generation has a CAR molecule formed by fusing the signaling domain of a CD3ζ chain or FcεRIγ and the antigen recognition region, and comprising no costimulatory domains. The first-generation CAR-T has limited proliferation ability *in vivo* and is prone to apoptosis. The second-generation CAR is added with a costimulatory domain, such as CD28 or 4-1BB(CD137). CD28 has strong anti-tumor activity, and the advantage of 4-1BB is that it can prolong the survival time of T lymphocytes and maintain its anti-tumor effects. The second-generation CAR has stronger proliferation ability than the first-generation CAR, and can secrete more cytokines and anti-apoptotic proteins. The third-generation CAR-T can not only express two costimulatory signal molecules at the same time, but also secrete more IFN-γ, and has stronger anti-tumor cytotoxicity. The fourth-generation CAR-T can further secrete specific cytokines(such as IL-12) in tumors, thereby changing the tumor microenvironment, and influencing and activating other immune cells to generate an immune response.

As used herein, the "signal peptide" refers to a short peptide chain that guides the transfer of newly synthesized proteins to the secretory pathway, usually 5-30 amino acids in length. In some embodiments, the signal peptide is a membrane localization signal peptide, i.e., an amino acid sequence used to direct transmembrane transfer(localization) of a protein. In most embodiments, the signal peptide is located at the N-terminal of the amino acid sequence. In mRNA, the coding sequence of the signal peptide is usually located after the initiation codon, which is an RNA region encoding a hydrophobic amino acid sequence. After the signal peptide guides the protein to complete its positioning, it is usually excised under the action of signal peptidase.

As used herein, a "variant" of the protein or nucleic acid refers to a protein or nucleic acid that has the same function but has one or more mutations in its sequence as compared with a specific protein or nucleic acid. For example, a "variant" of a certain protein refers to a protein having the same function and at least 70% sequence identity with the certain protein, obtained by artificial or natural mutations which lead to mutations such as insertions, deletions, and substations of one or more amino acids in the amino acid sequence of the certain protein.

"Identity" of sequences as used herein refers to the degree of similarity between amino acid sequences or between nucleotide sequences as determined by sequence alignment software, such as BLAST.

Those skilled in the art should know that the imbalance of BAFF-BAFF-R signaling pathway can cause various diseases, including autoimmune diseases, graft-versus-host diseases and tumors. The neoplasms include all B-cell malignancies except plasma cell lesions(multiple myeloma), such as mantle cell lymphoma, follicular lymphoma, diffuse large B-cell lymphoma, marginal zone lymphoma, Burkitt's Lymphoma, lymphoblastic leukemia, chronic lymphocytic leukemia, hairy cell leukemia, etc. The autoimmune diseases include, for example, rheumatoid arthritis, systemic lupus erythematosus, multiple sclerosis, glomerular nephritis, Sjögren's syndrome, or autoimmune hemolytic anemia.

As used herein, the term "effective amount" or "therapeutically effective amount" refers to the amount of a pharmaceutical composition comprising one or more peptides, proteins, nucleic acids or mutants, variants, analogs or derivatives thereof disclosed in the present application, and the patient or subject receiving the "effective amount" or "therapeutically effective amount" of the pharmaceutical composition can obtain a reasonable benefit/risk ratio of medical treatment, thereby alleviating or preventing at least one or more of symptoms of a disease or a condition and achieving the desired therapeutic or preventive effect.

As used herein, the term "about" refers to the usual error range for the respective value readily known to those skilled in the art. Reference to "about" a value or parameter herein includes(and describes) embodiments that are directed to that value or parameter per se. As used herein, the term "about" when preceding a numerical value means within 10% above or below the numerical value. For example, "about 100" encompasses 90 and 110.

As used herein, the singular forms "a", "an" and "the" include plural forms unless stated otherwise.

Unless otherwise defined herein, all technical and scientific terms used herein have the same meaning as commonly understood by one of ordinary skill in the art to which this invention belongs.

Chimeric antigen receptor

The present application provides a chimeric antigen receptor(CAR) specifically recognizing BAFF-R..

The chimeric antigen receptor(CAR)specifically recognizing BAFF-R comprises a specific recognition domain and an activation stimulation domain, and a transmembrane domain located between the specific recognition domain and activation stimulation domain, wherein, the specific recognition domain refers to the part of the CAR that specifically binds the target antigen on a target cell. In some embodiments, the specific recognition domain comprises an antibody or a functional equivalent thereof or a fragment or derivative thereof, for example may comprise a full-length heavy chain, a Fab fragment, a single chain Fv(scFv) fragment, a bivalent single-chain antibody or a diabody, each specific for a target antigen. However, many alternatives also exist, such as cytokines(which recognize cells bearing cytokine receptors), affibodies, ligand binding domains from naturally occurring receptors, soluble protein/peptide ligands for receptors (for example, on tumor cells), etc., each of which can be used in various embodiments of the present invention. Those skilled in the art shall understand that virtually any molecule or domain that binds the target antigen with high affinity can be used as a specific recognition domain.

The heavy chain variable region of the chimeric antigen receptor(CAR) specifically recognizing BAFF-R according to the invention comprises three complementarity determining regions, respectively CDR H1, CDR H2 and CDR H3, and the CDR H1, CDR H2 and CDR H3 respectively comprise amino acid sequence SEQ ID NO: 6, SEQ ID NO: 7, and SEQ ID NO: 8; and the light chain variable region also comprises three complementarity determining regions, respectively CDR L1, CDR L2 and CDR L3, and the CDR L1, CDR L2 and CDR L3 respectively comprise amino acid sequence SEQ ID NO: 10, SEQ ID NO: 11, and SEQ ID NO: 12.

In some embodiments, the heavy chain variable region and the light chain variable region further comprise a framework region, preferably a human antibody framework region.

In some embodiments, the heavy chain variable region comprises an amino acid sequence set forth in SEQ ID NO: 5 or an amino acid sequence having from 80% to 100% sequence identity therewith; and the light chain variable region comprises an amino acid sequence set forth in SEQ ID NO: 9 or an amino acid sequence having from 80% to 100% sequence identity therewith.

In some embodiments, the heavy chain variable region and the light chain variable region are linked by a linker to form an scFv, and the scFv comprises an amino acid sequence set forth in SEQ ID NO: 4 or an amino acid sequence having

EP 4 342 913 B1

about or more than 70% sequence identity therewith, for example, an amino acid sequence having about 75%, 80%, 85%, 90%, 95%, 97%, 98%, or 99% or more sequence identity therewith.

A CAR usually comprises a specific recognition domain, an activation stimulation domain, and a transmembrane domain between them. There are three conventional CAR structures, the difference mainly lies in the difference in the activation stimulation domain, including:

the first-generation CARs whose activation stimulation domain comprises an intracellular signaling domain but lacks the costimulatory domain;

the second-generation CARs, whose activation stimulation domain comprises an intracellular signaling domain and one costimulatory domain;

the third-generation CARs, whose activation stimulation domain comprises an intracellular signaling domain and two costimulatory domains.

Accordingly, in some embodiments of the present application, the CAR is a first-generation CAR in which the signaling domain can be cytoplasmic and can transduce effector function signals and direct the cell to perform its specialized functions. Examples of such signaling domains include, but are not limited to, the $\zeta$ chain of a T cell receptor or any homologues thereof(e.g., $\eta$ chain, Fc$\epsilon$R1$\gamma$, $\beta$ chain, MB1(Ig$\alpha$) chain, B29(Ig$\beta$) chain, etc.), CD3 polypeptides($\gamma$, $\delta$, and $\epsilon$), syk family tyrosine kinases(Syk, ZAP70, etc.), src family tyrosine kinases(Lck, Fyn, Lyn, etc.) and other molecules involved in T cell transduction, such as CD2, CD5 and CD28. Specifically, the intracellular signaling domain can be a human CD3$\zeta$ chain, Fc$\gamma$RIII, Fc$\epsilon$RI, the cytoplasmic tail of an Fc receptor, a cytoplasmic receptor with an immunoreceptor tyrosine activation motif(ITAM), or a combination thereof. Other intracellular signaling domains will be apparent to those skilled in the art and may be used in combination with alternative embodiments of the invention. In some embodiments, the signaling domain comprised by the activation stimulation domain is a signaling domain comprising an immunoreceptor tyrosine activation motif. In some embodiments, the intracellular signaling domain comprises a signaling domain of one or more selected from the group consisting of: intracellular regions of CD3$\zeta$, CD3$\gamma$, CD3$\delta$, CD3$\epsilon$, Fc$\epsilon$RI$\gamma$, Fc$\epsilon$R1$\beta$, CD79$\alpha$, CD79$\beta$, Fc$\gamma$RIIa, DAP10 and DAP12 molecules, or variants retaining the same function thereof. In some preferred embodiments, the intracellular signaling domain comprises the intracellular region of CD3$\zeta$ or Fc$\epsilon$RI$\gamma$, or the variants retaining the same function thereof. In some preferred embodiments, the intracellular signaling domain is derived from the intracellular region of CD3$\zeta$. In some embodiments, the intracellular signaling domain comprises an amino acid sequence set forth in SEQ ID NO: 20 or an amino acid sequence having about 70% or more sequence identity therewith, for example having an amino acid sequence having about 75%, 80% , 85%, 90%, 95%, 97%, 98%, or 99% or more sequence identity therewith.

In some embodiments of the present application, the CAR is a second-generation CAR, wherein the signaling domain is the above-mentioned signaling domain, and the costimulatory domain further comprised is any one of the intracellular regions of the following molecules: CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80(KLRF1), CD160, CD19, CD4, CD8$\alpha$, CD8$\beta$, IL2R$\beta$, IL2R$\gamma$, IL7R$\alpha$, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-I, ITGB7, TNFR2, TRANCE/RANKL, DNAMI, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D and ligands specifically binding CD83, or variants retaining the same function thereof.

In some embodiments of the present application, the CAR is a third-generation CAR, wherein the signaling domain is the above-mentioned signaling domain, and the costimulatory domain further included is any two, three, or more of the intracellular regions of the following molecules: CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80(KLRF1), CD160, CD19, CD4, CD8$\alpha$, CD8$\beta$, IL2R$\beta$, IL2R$\gamma$, IL7R$\alpha$, ITGA4, VLA1, CD49a, ITGA4, IA4, CD49D, ITGA6, VLA -6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 , SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and ligands specifically binding CD83, or variants retaining the same functions thereof.

In some preferred embodiments, the costimulatory domain is preferably the 4-1BB intracellular region, the CD28 intracellular region, the OX40 intracellular region, or variants retaining the same functions thereof. In some specific embodiments, the costimulatory domain comprises an intracellular region derived from 4-1BB. In some specific embodiments, the costimulatory domain comprises the amino acid sequence of the human 4-1BB molecular(4-1BB) intracellular region set forth in SEQ ID NO: 18 or an amino acid sequence having from 80% to 100% sequence identity therewith.

Those skilled in the art shall know that through the combination of the signaling domain and the costimulatory domain in an appropriate manner, the effector cells where the CAR is located can get appropriate activation signal when

9

the CAR binds to the ligand.

In some embodiments, the transmembrane domain between the specific recognition domain and the activation stimulation domain can be derived from any transmembrane sequence of any proteins with a transmembrane domain(including any of type I, type II or type III transmembrane proteins). In addition, the transmembrane domain of the CAR of the present invention may also contain artificial hydrophobic sequences. In some embodiments, the transmembrane domain comprises the transmembrane domain of a molecule selected from the group consisting of: TCRα, TCRβ, TCRγ, CD3ζ, CD3ε, CD45, CD4, CD5, CD8, CD9, CD16, CD22, CD28, CD33, CD37, CD64, CD80, CD86, CD134, CD137, CD154, KIRDS2, OX40, CD2, CD27, LFA-1, ICOS(CD278), 4-1BB, CTLA-4, GITR, CD40, BAFFR, LIGHTR, SLAMF7 , NKp80, CD160, CD19, IL2Rβ, IL2Rγ, IL7Rα, ITGA1, VLA1, CD49α, ITGA4, IA4, CD49D, ITGA6, VLA-6, CD49f, ITGAD, CD11d, ITGAE, CD103, ITGAL, CD11a, ITGAM, CD11b, ITGAX , CD11c, ITGB1, CD29, ITGB2, CD18, ITGB7, TNFR2, CD226, SLAMF4, CD84, CD96, CEACAM1, CRT AM, CD229, CD160, PSGL1, CDIOO, SLAMF6, SLAMF1, SLAMF8, CD162, LTBR, PAG/Cbp, NKp44, NKp30, NKp46, NKG2D, or NKG2C; preferably the transmembrane domain of CD4, CD8α, CD28, CD3ζ, PD1, or 4-1BB or a variant with the same function thereof.

In some embodiments, the transmembrane domain may comprise one or more additional amino acids adjacent to a transmembrane domain, for example, one or more(e.g., 1, 2, 3, 4, 5, 6, 7, 8, 9, 10 to 15 amino acids of the extracellular region) amino acids associated with the extracellular region of the protein from which the transmembrane domain is derived and/or with the one or more(e.g., 1, 2, 3, 4, 5, 6, 7, 8, 9, 10 to 15 amino acids of an intracellular region) additional amino acids associated with the intracellular region of the protein from which the transmembrane domain is derived. In one aspect, the transmembrane domain is derived from the same protein as the signaling domain, costimulatory domain or hinge domain is derived from. In another aspect, the transmembrane domain is not derived from the same protein as any other domain of the CAR is derived from. In some preferred embodiments of the present application, the transmembrane domain comprises the transmembrane domain of CD8α. In some embodiments, the transmembrane domain comprises an amino acid sequence set forth in SEQ ID NO: 16 or an amino acid sequence having about 70% or more sequence identity therewith, such as an amino acid sequence having about 75%, 80%, 85%, 90%, 95%, 97%, 98%, 99% or more sequence identity therewith.

In some embodiments, the transmembrane domain is directly linked to the specific recognition domain. In some embodiments, the transmembrane domain and the specific recognition domain are linked by a hinge region. In some embodiments, the hinge region comprises an amino acid sequence selected from the group consisting of: an Fc fragment of human CD8α or an antibody or a functional equivalent, fragment or derivative thereof, a hinge region of human CD8α or an antibody or a functional equivalent, fragment or derivative thereof, CH2 region of an antibody, CH3 region of an antibody, artificial spacer sequence and a combination thereof; preferably the hinge region amino acid sequence of IgG, IgD, CD8α or CD28. In some exemplary embodiments, the hinge region comprises any one or more of:(i) an IgG4 hinge region, a CH2 region, and a CH3 region,(ii) an IgG4 hinge region,(iii) an IgG4 hinge region and a CH2 region,(iv) a CD8α hinge region,(v) an IgG1 hinge region, a CH2 region and a CH3 region,(vi) an IgG1 hinge region,(vi) an IgG1 hinge region and a CH2 region, or(vii) a combination thereof. In some specific embodiments, the hinge region is the hinge region of a human CD8α. In some specific embodiments, the hinge region comprises an amino acid sequence set forth in SEQ ID NO: 14 or an amino acid sequence having about 70% or more sequence identity therewith, such as an amino acid sequence having about 75%, 80%, 85%, 90%, 95%, 97%, 98%or 99% or more sequence identity therewith.

In some embodiments, the CAR further comprises a signal peptide. Usually the signal peptide(SP) is located at the N-terminal of the protein and carries information of protein secretion, which participates in determining the secretion pathway and distribution of the protein. Those skilled in the art should know that proteins located in the nucleus, mitochondria, and cytoplasm, as well as free proteins in the cytoplasm and proteins secreted or finally anchored on the cell membrane usually have different signal peptides. Those skilled in the art can select a signal peptide sequence suitable for a certain protein to function through software prediction and signal peptide database. Therefore, in some embodiments of the present application, the signal peptide is selected from signal peptide sequences of any secreted protein or cell membrane protein, or variants retaining the same functions thereof. In some embodiments, the signal peptide sequence is the signal peptide of CD8α. In some embodiments, the signal peptide sequence comprises an amino acid sequence such as SEQ ID NO: 2 or an amino acid sequence having about 70% or more sequence identity therewith, for example having about 75%, 80%, 85%, 90%, 95%, 97%, 98%, or 99% or more sequence identity therewith.

Nucleic acid molecule

The present application also provides a nucleic acid molecule encoding a receptor specifically recognizing BAFF-R or a fragment thereof, wherein the receptor specifically recognizing BAFF-R is the aforementioned chimeric antigen receptor. The nucleic acid molecule comprises a polynucleotide sequence encoding the aforementioned chimeric antigen receptor or a fragment thereof. The nucleic acid molecule can be DNA or RNA. In some embodiments, the nucleic acid molecule is linear, and in some embodiments, the nucleic acid molecule is circular. In some embodiments, the nucleic acid molecule is double stranded. In some embodiments, the nucleic acid molecule is single stranded. In some embodiments, the nucleic acid molecule is chemically synthesized. In some embodiments, the nucleic acid molecule comprises chemical modifications to render it more stable in the cell or in the animal body. In some embodiments, the nucleic acid molecule is synthesized by bacterial, fungal, or animal cells. In some embodiments, the nucleic acid molecule is a plasmid, viral vector, or oligonucleotide.

The polynucleotide sequence comprises a specific recognition domain encoding the receptor specifically recognizing BAFF-R. In some embodiments, the specific recognition domain is a scFv. In some embodiments, the polynucleotide sequence comprises a DNA sequence set forth in SEQ ID NO: 3 and/or a DNA complementary thereto, or an RNA sequence corresponding or complementary thereto, or a polynucleotide sequence having from 80% to 100% sequence identity to the DNA or RNA sequence described in this paragraph. As used herein, the "complementary" of nucleic acids means that two polynucleotides can hybridize under stringent conditions, and stringent hybridization means that when the two polynucleotides hybridize, each base or each nucleotide are all paired in accordance with the Watson-Crick base pairing principle, that is, A is paired with T or U, and C is paired with G or I. As used herein, the RNA "corresponding" to a DNA sequence refers to a ribonucleotide(RNA) sequence that is consistent with the sequence of bases in the DNA sequence and in which all T bases are replaced by U bases.

In some embodiments, the nucleic acid molecule comprises a coding sequence of a membrane localization signal peptide molecule. In some embodiments, the membrane localization signal peptide is a membrane localization signal peptide of a CD8α. In some embodiments, the coding sequence of the membrane localization signal peptide molecule comprises the DNA sequence set forth in SEQ ID NO: 1 and/or the DNA complementary thereto, or the RNA sequence corresponding or complementary thereto, or a polynucleotide sequence having about 70% or more sequence identity with the DNA or RNA sequence described in this paragraph, for example a polynucleotide sequence having about 75%, 80%, 85%, 90%, 95%, 97%, 98%, or 99% or more sequence identity therewith.

In some embodiments, the nucleic acid molecule comprises a sequence encoding a hinge region. In some embodiments, the hinge region being encoded is the hinge region of a CD8α. In some embodiments, the sequence encoding the hinge region comprises a DNA sequence set forth in SEQ ID NO: 13 and/or DNA complementary thereto, or an RNA sequence corresponding or complementary thereto, or a polynucleotide sequence having about 70% or more sequence identity to a DNA or RNA sequence as described in this paragraph, for example a polynucleotide sequence having about 75%, 80%, 85%, 90%, 95%, 97%, 98%, or 99% or more sequence identity therewith.

In some embodiments, the nucleic acid molecule comprises a sequence encoding a signaling domain. In some embodiments, the signaling domain is the signaling domain of a CD3ζ. In some embodiments, the sequence encoding the signaling domain comprises a DNA sequence set forth in SEQ ID NO: 19 and/or a DNA complementary thereto, or an RNA sequence corresponding or complementary thereto, or a polynucleotide sequence having about 70% or more sequence identity to a DNA or RNA sequence as described in this paragraph, for example a polynucleotide sequence having about 75%, 80%, 85%, 90%, 95%, 97%, 98%, or 99% or more sequence identity therewith.

In some embodiments, the nucleic acid molecule comprises a sequence encoding a costimulatory domain. In some embodiments, the costimulatory domain is the signaling domain of a 4-1BB. In some embodiments, the sequence encoding the signaling domain comprises a DNA sequence set forth in SEQ ID NO: 17 and/or a DNA complementary thereto, or an RNA sequence corresponding or complementary thereto, or a polynucleotide sequence having about 70% or more sequence identity to a DNA or RNA sequence as described in this paragraph, for example a polynucleotide sequence having about 75%, 80%, 85%, 90%, 95%, 97%, 98%, or 99% or more sequence identity therewith.

In some embodiments, the nucleic acid molecule comprises the aforementioned sequence encoding the costimulatory domain, the aforementioned sequence encoding the signaling domain, the aforementioned sequence encoding the hinge region, the aforementioned sequence encoding the membrane localization signal peptide molecule, and the aforementioned sequence encoding the specific recognition domain. How to arrange the sequence comprised in the aforementioned nucleic acid molecule to make the nucleic acid molecule, and which related components to regulate expression to be added, so that after the nucleic acid molecule is introduced into the cell, and a complete receptor that specifically recognizes BAFF-R can be successfully synthesized is obvious to those skilled in the art. In some specific embodiments, the nucleic acid molecule sequentially comprises the polynucleotide sequences described below from the 5' end to the 3' end or polynucleotide sequences having from 80% to 100% sequence identity therewith: SEQ ID NO: 1, SEQ ID NO: 3, SEQ ID NO: 13, SEQ ID NO: 15, SEQ ID NO: 17 and SEQ ID NO: 19, wherein other sequences may be comprised between the polynucleotide sequences.

The present application also provides a mixture comprising multiple nucleic acid molecules. The multiple nucleic acid molecules respectively comprise one or more of the following: the aforementioned sequence encoding the costimulatory domain, the aforementioned sequence encoding the signaling domain, the aforementioned sequence encoding the hinge region, the aforementioned sequence encoding the membrane localization signal peptide molecule, the aforementioned sequence encoding the specific recognition domain. The mixture is co-introduced into a cell, and then a complete receptor specifically recognizing BAFF-R can be synthesized.

## Engineered immune effector cells

An engineered immune effector cell, comprising the aforementioned receptor specifically recognizing BAFF-R or the aforementioned nucleic acid molecule.

In some embodiments, the receptor specifically recognizing BAFF-R is the aforementioned chimeric antigen receptor, and the engineered immune effector cell is a CAR-T or CAR-NK cell.

## Virus particle, liposome, lipid nanoparticle, and pharmaceutical composition

The present application also provides a viral plasmid, wherein the packaged viral vector comprises the aforementioned chimeric antigen receptor and/or encodes the chimeric antigen receptor polynucleotide sequence. The present application also provides a liposome or a lipid nanoparticle, wherein the liposome or lipid nanoparticle comprises the aforementioned receptor specifically recognizing BAFF-R a or a fragment thereof or a nucleic acid molecule encoding the receptor or the fragment thereof.

The present application further provides a pharmaceutical composition, which comprises a therapeutically effective amount of one or more selected from the group consisting of: the aforementioned chimeric antigen receptor, the aforementioned nucleic acid molecule, the aforementioned engineered immune effector cell, the aforementioned viral particle, liposome, and lipid nanoparticle. In some embodiments, the pharmaceutical composition further comprises a suitable excipient, a carrier, and/or a stabilizer. The acceptable carrier, excipient or stabilizer is nontoxic to recipients at the dosages and concentrations employed, and comprises, for example, a buffer such as phosphate, citrate or acetate, usually at a pH of 5.0 to 8.0, optionally 6.0 to 7.0; a salt to achieve isotonicity, such as a sodium chloride, potassium chloride, etc.; an antioxidant; a preservative; a low molecular weight polypeptide; a proteins; a hydrophilic polymer, such as a polysorbate 80; an amino acid, such as a glycine; a carbohydrate; a chelating agent; a sugar; and other standard ingredients known to those skilled in the art(Remington: The Science and Practice of Pharmacy, 22nd Ed., Loyd V. Allen et al. eds. Pharmaceutical Press(2012)).

## Therapy

The present application further discloses a method for treating or preventing a disease or symptom of a subject in need, the method comprising administering to the subject an effective amount of the aforementioned pharmaceutical composition, and the disease being selected from diseases caused by imbalances of the cellular BAFF-BAFF-R signaling pathway. Optionally, the method further comprises administering to the subject a second therapeutic agent, such as a monoclonal antibody capable of binding to the antigen CD20, a monoclonal antibody capable of binding to CD19, a monoclonal antibody capable of binding to an immune checkpoint, such as PD-1/PD-L1, or an engineered immune effector cell capable of targeting CD20, CD19, or PD-L1, etc. In some embodiments, the disease is any one selected from the group consisting of: autoimmune diseases, graft versus host diseases, or tumors.

In some embodiments, the disease is a cancer. Optionally, the cancer is a lymphoma, leukemia or myeloma. Optionally, the cancer is a lymphoma. Optionally, the lymphoma is a mantle cell lymphoma, follicular lymphoma, diffuse large B-cell lymphoma, marginal zone lymphoma, or Burkitt's lymphoma. Optionally, the cancer is a leukemia. Optionally, the leukemia is a lymphoblastic leukemia, chronic lymphocytic leukemia or hairy cell leukemia. Optionally, the cancer is a myeloma. Optionally, the myeloma is a multiple myeloma.

In some embodiments, the disease is an autoimmune disease. Optionally, the autoimmune disease is a rheumatoid arthritis, systemic lupus erythematosus, multiple sclerosis, glomerulonephritis, Sjögren's syndrome, or autoimmune hemolytic anemia.

In another aspect, the present application also provides a method for inhibiting cell proliferation. The method comprises contacting the cells with a pharmaceutical composition as provided herein, thereby forming contacted cells. The anti-BAFF-R receptor or its functional fragments are combined with the BAFF-R protein on the cells to inhibit cell proliferation. Optionally, the cells are lymphocytes. Optionally, the cells are B cells or cancer cells. Optionally, the cells are lymphoma cells.

As used herein, treating or preventing a disease or condition refers to methods of obtaining beneficial or desired results, including clinical results. Beneficial or desired clinical results may include, but are not limited to: alleviation or amelioration of one or more symptoms or conditions; lessening the extent of a condition, disorder or disease; stabilizing the state of a condition, disorder or disease; preventing the development of a condition, disorder or disease; preventing the spread of a condition, disorder or disease; delaying or slowing the progression of a condition, disorder or disease; delaying or slowing the onset of a condition, disorder or disease; ameliorating or palliating of a condition, disorder or disease state; or turning better. Treating can also mean prolonging the survival of a subject beyond expected survival in the absence of treatment. Treating can also mean inhibiting the progression of a condition, disorder or disease, temporarily slowing the progression of a condition, disorder or disease, but in some cases involving permanently stopping the progression of a condition, disorder or disease.

**EXAMPLE**

**Example 1. Design of chimeric antigen receptors targeting BAFF-R**

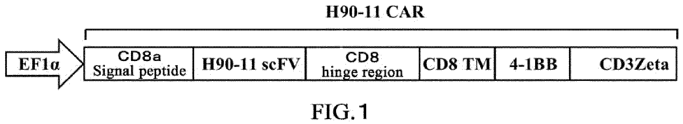

The present invention constructs an anti-BAFF-R chimeric antigen receptor(H90-11 CAR, which comprises the heavy chain variable region and light chain variable region of the H90-11 mAb monoclonal antibody shown by the disclosure of the US application: US 2021/0261676AI, as shown in the schematic diagram of the structure in FIG. 1, the chimeric antigen receptor comprises a signal peptide sequence(Leader) of a CD8α, a single-chain antibody sequence(scFv) specifically binding to the BAFF-R antigen, a hinge region(Hinge) and a transmembrane domain sequence(Transmembrane) of a human CD8α, and a 4-1BB costimulatory domain sequence and a CD3ζ signaling domain sequence. The specific sequence of each part is as follows, and the specific sequence is shown in Table 1:

The polynucleotide sequence of the human CD8α molecular signal peptide(Leading signal) is set forth in SEQ ID NO: 1, and the amino acid sequence is set forth in SEQ ID NO: 2;

The polynucleotide sequence of the human BAFF-R single-chain antibody(scFv) is set forth in SEQ ID NO: 3, and the amino acid sequence is set forth in SEQ ID NO: 4; wherein the heavy chain variable region(VH) of the scFv is SEQ ID NO:5, the amino acid sequences of the three complementary determining regions CDR H1, CDR H2, and CDR H3 comprised in the VH are respectively: SEQ ID NO:6, SEQ ID NO:7, and SEQ ID NO:8; the light chain variable region(VL) of the scFv is SEQ ID NO: 9, and the amino acid sequences of the three complementary determining regions CDR L1, CDR L2, and CDR L3 comprised in the VL are: SEQ ID NO: 10, SEQ ID NO : 11, and SEQ ID NO: 12;

The polynucleotide sequence of the human CD8α hinge region(CD8 Hinge) is set forth in SEQ ID NO: 13, and the amino acid sequence is set forth in SEQ ID NO: 14;

The polynucleotide sequence of the human CD8α transmembrane domain(CD8TM) is set forth in SEQ ID NO: 15, and the amino acid sequence is set forth in SEQ ID NO: 16;

The polynucleotide sequence of the human 4-1BB molecular(4-1BB) intracellular region is set forth in SEQ ID NO: 17, and the amino acid sequence is set forth in SEQ ID NO: 18;

The nucleotide sequence of the human CD3ζ molecular(CD3ζ) intracellular region is set forth in SEQ ID NO: 19, and the amino acid sequence is set forth in SEQ ID NO: 20;

**Table 1.**

| SEQ ID NO. | Specific sequence (5' end to 3' end/N-terminal to C-terminal) |
|---|---|
| SEQ ID NO: 1 | ATGGCCCTCCCTGTCACCGCCCTGCTGCTTCCGCTGGCTCT TCTGCTCCACGCCGCTCGGCCC |
| SEQ ID NO: 2 | MALPVTALLLPLALLLHAARP |
| SEQ ID NO: 3 | GACATCCAGATGACACAGAGCCCTGATAGCCTGGCCGTGTC |

(continued)

| SEQ ID NO. | Specific sequence (5' end to 3' end/N-terminal to C-terminal) |
|---|---|
| | TCTGGGAGAGAGAGCCACCATCAACTGCAAGAGCAGCCAG AGCGTGCTGTACTCCAGCAACAACAAGAACTACCTGGCCT GGTATCAGCAGAAGCCCGGCCAGCCTCCTAAGCTGCTGATC TACTGGGCCAGCATCAGAGAAAGCGGCGTGCCCGATAGATT TTCCGGCTCTGGCAGCGGCACCGACTTCACCCTGACCATAT CTAGCCTGCAGCCTGAGGACTTCGCCACCTACTACTGCCAG CAGAGCTACAGCACCCCTTGGACATTTGGCCAGGGCACCA AGCTGGAAATCAAGGGAGGCGGAGGTTCTGGCGGCGGAG GAAGTGGTGGCGGAGGCTCTGAGGTTCAGCTGCAAGAGTC TGGCCCTGGCCTGGTCAAGCCTAGCCAAACACTGAGCCTG ACCTGTACCGTGTCCGGCGATAGCATCACAAGCGGCTACTG GAACTGGATCAGACAGCACCCTGGCAAGGGCCTCGAGTAC ATCGGCTACATCAGCTACAGCGGCAGCACCTACTACAACCC CAGCCTGAAGTCCAGAGTGACCATCAGCAGAGACACCAGC AAGAACCAGTACTCCCTGAAGCTGAGCAGCGTGACAGCCG CCGATACAGCCGTGTACTACTGCGCCTCTCCTAACTACCCT TCTACGCCATGGACTACTGGGGCCAGGGAACCCTGGTTACC GTTAGCTCT |
| SEQ ID NO: 4 | DIQMTQSPDSLAVSLGERATINCKSSQSVLYSSNNKNYLAWY QQKPGQPPKLLIYWASIRESGVPDRFSGSGSGTDFTLTISSLQP EDFATYYCQQSYSTPWTFGQGTKLEIKGGGGSGGGGSGGGGS EVQLQESGPGLVKPSQTLSLTCTVSGDSITSGYWNWIRQHPGK GLEYIGYISYSGSTYYNPSLKSRVTISRDTSKNQYSLKLSSVTA ADTAVYYCASPNYPFYAMDYWGQGTLVTVSS |
| SEQ ID NO: 5 | EVQLQESGPGLVKPSQTLSLTCTVSGDSITSGYWNWIRQHPGK GLEYIGYISYSGSTYYNPSLKSRVTISRDTSKNQYSLKLSSVTA ADTAVYYCASPNYPFYAMDYWGQGTLVTVSS |
| SEQ ID NO: 6 | SGYWN |
| SEQ ID NO: 7 | YISYSGSTYYNPSLKS |
| SEQ ID NO: 8 | PNYPFYAMDY |
| SEQ ID NO: 9 | DIQMTQSPDSLAVSLGERATINCKSSQSVLYSSNNKNYLAWY QQKPGQPPKLLIYWASIRESGVPDRFSGSGSGTDFTLTISSLQP EDFATYYCQQSYSTPWTFGQGTKLEIK |
| SEQ ID NO: 10 | KSSQSVLYSSNNKNYLA |
| SEQ ID NO: 11 | WASIRES |
| SEQ ID NO: 12 | QQSYSTPWT |
| SEQ ID NO: 13 | ACCACGACGCCAGCGCCGCGACCACCAACACCGGCGCCCA CCATCGCGTCGCAGCCCCTGTCCCTGCGCCCAGAAGCGTGC CGGCCAGCGGCGGGGGGCGCAGTGCACACGAGGGGGCTG GACTTCGCCTGTGAT |

(continued)

| SEQ ID NO. | Specific sequence (5' end to 3' end/N-terminal to C-terminal) |
|---|---|
| SEQ ID NO: 14 | TTTPAPRPPTPAPTIASQPLSLRPEACRPAAGGAVHTRGLDFACD |
| SEQ ID NO: 15 | ATCTACATTTGGGCCCCTCTGGCTGGTACTTGCGGGGTCCT |
| SEQ ID NO: 16 | GCTGCTTTCACTCGTGATCACTCTTTACTGT |
| | IYIWAPLAGTCGVLLLSLVITLYC |
| SEQ ID NO: 17 | AAGCGCGGTCGGAAGAAGCTGCTGTACATCTTTAAGCAAC CCTTCATGAGGCCTGTGCAGACTACTCAAGAGGAGGACGG CTGTTCATGCCGGTTCCCAGAGGAGGAGGAAGGCGGCTGC GAACTG |
| SEQ ID NO: 18 | KRGRKKLLYIFKQPFMRPVQTTQEEDGCSCRFPEEEEGGCEL |
| SEQ ID NO: 19 | CGCGTGAAATTCAGCCGCAGCGCAGATGCTCCAGCCTACA AGCAGGGGCAGAACCAGCTCTACAACGAACTCAATCTTGG TCGGAGAGAGGAGTACGACGTGCTGGACAAGCGGAGAGG ACGGGACCCAGAAATGGGCGGGAAGCCGCGCAGAAAGAA TCCCCAAGAGGGCCTGTACAACGAGCTCCAAAAGGATAAG ATGGCAGAAGCCTATAGCGAGATTGGTATGAAAGGGGAAC GCAGAAGAGGCAAAGGCCACGACGGACTGTACCAGGGAC TCAGCACCGCCACCAAGGACACCTATGACGCTCTTCACATG CAGGCCCTGCCGCCTCGG |
| SEQ ID NO: 20 | RVKFSRSADAPAYKQGQNQLYNELNLGRREEYDVLDKRRGR DPEMGGKPRRKNPQEGLYNELQKDKMAEAYSEIGMKGERRR GKGHDGLYQGLSTATKDTYDALHMQALPPR |

## Example 2 Construction of Chimeric Antigen Receptor Expression Vector

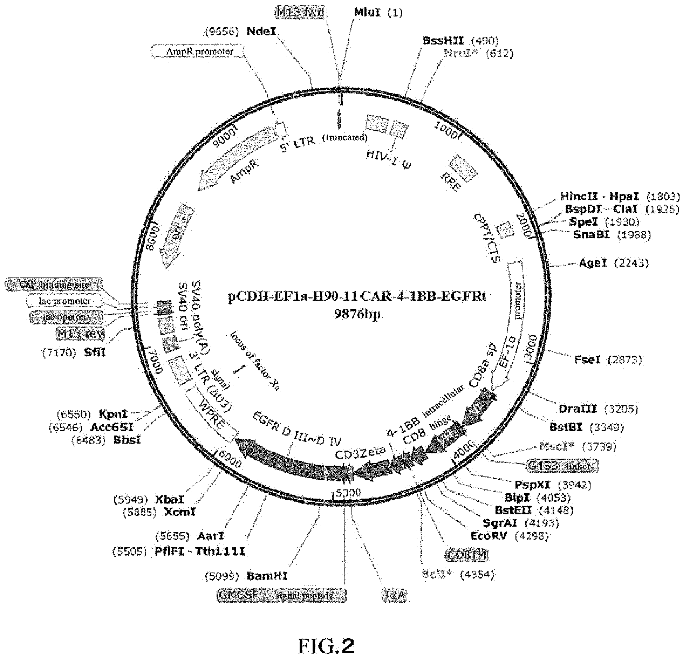

The anti-BAFF-R H90-11 CAR lentiviral expression vector pCDH-EF1a-H90-11CAR-4-1BB-EGFRt and the control EGFRt CAR lentiviral expression vector pCDH-EF1a-EGFRt-AT-Free(delete T2A) were both synthesized and constructed by Nanjing GenScript Company, the specific experimental steps were as follows:
The anti-BAFF-R scFv gene sequence was synthesized by Nanjing GenScript Company, and the synthesized scFv gene sequence was inserted into the pCDH-EF1a-4-1BB-EGFRt vector(Nanjing GenScript, order number: C046EEE310), the anti-BAFF-R chimeric antigen receptor lentiviral expression vector pCDH-EF1a-H90-11CAR-4-1BB-EGFRt(Nanjing GenScript, order number: C9401FG210) was obtained, and the carrier map is shown in FIG. 2.

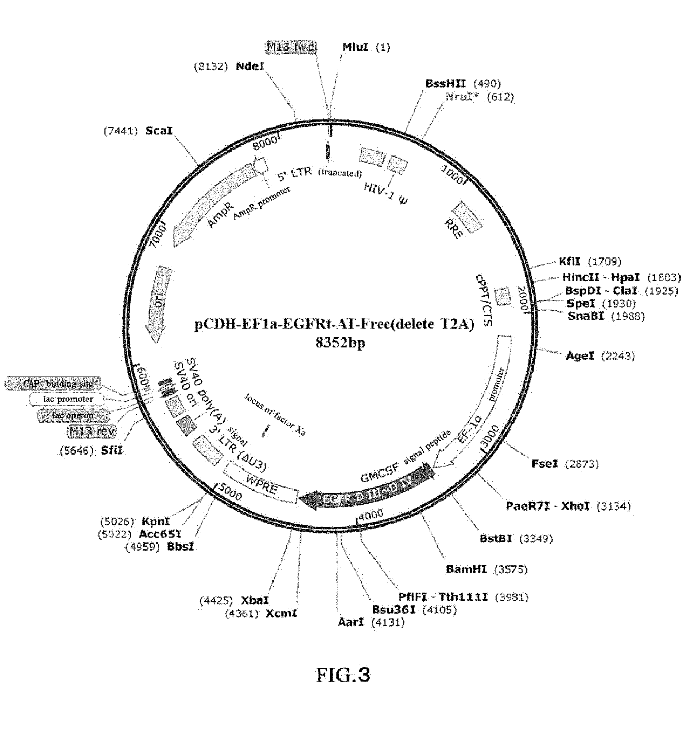

For pCDH-EF1a-T2A-EGFRt(provided by iCarTab) lentiviral expression vector, as there is an ATG start codon in the T2A linker, EGFRt cannot be expressed normally due to a frameshift mutation. According to the instruction manual of the commercial company of the In-Fusion Snap assembly seamless cloning reagent, T2A in the pCDH-EF1a-T2A-EGFRt vector was deleted to obtain the control EGFRt CAR lentiviral expression vector pCDH-EF1a-EGFRt-AT-Free(delete T2A) (constructed by Nanjing GenScript), vector map see FIG. 3.

## Example 3: Lentivirus packaging and titer determination

Lentivirus packaging

Preparing a 15cm culture dish, inoculating $1\times10^7$ 293T cells, adding DMEM(Gibco, Cat. No.: 41965-062) complete medium containing 10% FBS(Gibco, Cat. No.: 10099-141), placing the dish at 37°C, 5% $CO_2$ incubator and culturing overnight. Taking 2 mL of PBS(Gibco, Cat. No.: 14190-250) to one well of a 6-well plate, and adding 20 μg of lentiviral expression plasmid(pCDH-EF1a-H90-11 CAR-4-1BB-EGFRt or pCDH-EF1a-EGFRt-AT -Free(delete T2A)), 30 µL lentivirus packaging plasmid Mix(iCarTab, Cat. No.: LVP2MIX), pipetting up and down to mix well, adding 150 µL LVTransm transfection reagent(iCarTab, Cat. No.: LVTran100), immediately pipetting the Mix up and down, and placing it at room temperature for 10 minutes. Adding the aforementioned DNA/PEI complex dropwise into a 15 cm culture dish, shaking the culture dish gently, and mixing well. Placing the culture dish in a 37°C, 5% CO2 incubator, and after culturing for 6-8 hours, discarding the medium containing the transfection reagent and replacing it with fresh complete medium. After continuous cultivation for 48 hours, collecting the medium supernatant containing the virus in the culture dish, filtering it with a 0.45 µm filter membrane, and transferring it to a centrifuge tube, and centrifuging at 20,000 g at 4°C for 2 hours. After centrifugation, discarding the liquid in the centrifuge tube in a biological safety cabinet, adding 500 µL of PBS buffer to resuspend the virus pellet, and storing the virus in a -80°C refrigerator after aliquoting.

Virus titer determination

Thrawing 293T cells and adjusting the cell state to the logarithmic growth phase. Taking a new 6-well plate, inoculating 293T cells at $8 \times 10^5$ cells per well, supplementing the medium to a final volume of 2 mL, placing the 6-well plate in a 37°C, 5% $CO_2$ incubator, and culturing overnight. Adding the concentrated lentivirus to the aforementioned 6-well plate in an amount of 50 µL, and polybrene(Sigma, Cat. No.: 107689-10G) at a final concentration of 6 µg/mL at the same time, and returning the 6-well plate to a 37°C, 5% $CO_2$ incubator, and continually culturing for 48 hours.
After the culture, washing the cells in each well with PBS, and then extracting the genomic DNA using the MiniBEST Universal Genomic DNA Extraction Kit(Takara, Cat. No.: 9765), and determining the concentration of the extracted genomic DNA using NanoDrop2000. According to the PCR reaction system shown in Table 2 below, preparing the fluorescent quantitative PCR reaction premix.

Table 2:

| Reagent name | Volume (20µL reaction system) |
|---|---|
| 2×PCR Mix | 10 |
| Probe Mix(LTR probe, ALB probe) | 0.4 |
| Primer mix(LTR F&R, ALB F&R) | 0.4 |
| PCR grade water | 4.2 |

The sequences and fluorophores of the primers used are shown in Table 3 below.

Table 3:

| Primer name | Sequence message (5'-3') | Probe fluorophore |
|---|---|---|
| LTR F | TGACAGCCGCCTAGCATTTC(SEQ ID NO: 21) | |
| LTR R | GCTCGATATCAGCAGTTCTTGAAG(SEQ ID NO: 22) | |
| LTR Probe | CACGTGGCCCGAGAGCTGCATC(SEQ ID NO: 23) | 5'-FAM-BHQ1-3' |
| ALB F | GCTGTCATCTCTTGTGGGCTGT(SEQ ID NO: 24) | |
| ALB R | ACTCATGGGAGCTGCTGGTTC(SEQ ID NO: 25) | |
| ALB Probe | CCTGTCATGCCCACACAAATCTCTCC(SEQ ID NO: 26) | 5'-FAM-BHQ1-3' |

Taking the pUC-LTR(purchased from: iCarTab) and pUC-ALB plasmids(purchased from: iCarTab) out of the refrigerator, and diluting them by 10 times gradient dilution respectively for a total of 6 points to prepare the standard curve samples required for fluorescent quantitative PCR. Take a new 96-well PCR reaction plate, add 5 µL of genome samples or standard curve samples to each well, then add 15 µL of the PCR reaction master mix prepared above to each well, seal with a sealing film, and centrifuge briefly for 1 minute. PCR reactions were performed according to the PCR program in Table 4 below.

Table 4:

| | Temperature(°C) | Time(sec) |
|---|---|---|
| denaturation | 95 | 30 |
| 40 cycles for Step1,Step 2 | | |
| Step 1 | 95 | 5 |

(continued)

| 40 cycles for Step1,Step 2 | | |
|---|---|---|
| Step 2 | 60 | 30 |

After the PCR reaction, using the supporting software of Roche 480 to obtain the Ct value of the standard curve and the Ct value of the sample; taking the Ct value as the ordinate, and taking the Log value of the copy number as the abscissa to make a standard curve; calculating the copy number according to the standard curve. And calculating the titer of the virus according to the following formula:

$$\text{Lentivirus Titer (TU/mL)} = \frac{(\text{Copy}_{LTR} \div \text{Copy}_{ALB}) \times 2 \times \text{Cell}_{NO.}}{\text{Volume}_{virus}}(1)$$

The results of the lentivirus titer calculated according to the formula(1) are shown in Table 5 below.

Table 5:

| Virus name | Activity titer(TU/mL) |
|---|---|
| pCDH-EFIa-H90-11CAR-4-1BB-EGFRt Lentivirus | $1.77 \times 10^8$ |
| pCDH-EF1a-EGFRt-AT-Free(delete T2A) Lentivirus | $1.6 \times 10^8$ |

It can be seen from Table 5 that a high-titer lentivirus was packaged, which can be used to subsequently infect T cells to prepare CAR-T cells.

**Example 4 Preparation of CAR-T cells**

Isolation of T cells from peripheral blood PBMC

Transferring 10mL of anticoagulated peripheral blood sample to a 15mL sterile centrifuge tube, centrifuging at 800g for 20 minutes, removing the upper light yellow serum layer, then adding an equal volume of PBS to the lower peripheral blood cell layer, and gently inverting up and down to mix. Taking out the lymphocyte separation medium(Haoyang Biotechnology, Cat. No.: 1077), and inverting it up and down down several times to mix well. Taking another 15mL centrifuge tube, adding 5mL lymphocyte separation medium, slowly adding the diluted blood sample to the upper layer of the lymphocyte separation medium along the tube wall to avoid mixing the separation medium and blood sample, and centrifuging at 800g for 20 minutes. After centrifugation, gently taking out the centrifuge tube, pipetting the white mononuclear cell layer in the middle into a new sterile centrifuge tube, adding an equal volume of normal saline and mixing gently, centrifuging at 800g for 5 minutes, after centrifugation, removing the supernatant, washing the PBMCs again, adjusting the cell density to $5 \times 10^7$ cells/mL, and transfering 1 mL/tube to a 2 mL cell cryopreservation tube.

Washing Dynabeads® Human T-Expander CD3/CD28(Thermo, Cat. No.: 11141D) twice with PBS, adding an appropriate amount of Dynabeads® human T-Expander CD3/CD28 to the PBMC in a 2mL cell cryopreservation tube, mixing gently, and incubating at room temperature for 20 minutes. Inserting the 2mL cell cryopreservation tube into the magnetic pole, letting it stand at room temperature for 1 minute, inverting it gently, and pouring out the liquid in the tube. Taking the cryopreservation tube out of the magnetic pole, adding appropriate amount of X-Vivo 15 medium(containing 200IU/mL IL-2(Beijing Yuance Pharmaceutical Co., Ltd., Cat. No.: 20150713B), 10ng/mL IL-7(PrimeGene, Cat. No.: 101-07), 5ng/mL IL-15(PrimeGene, Cat. No.:101-15), resuspending and counting. Adjusting the cell concentration to $0.5 \sim 1 \times 10^6$ cells/ml, transferring them to 6 well plates, and placing them at 37°C, 5% CO2 incubator and continuously culturing for 48 hours.

Lentivirus infection of T cells

Adjusting the density of the cultured T cells to $1 \times 10^6$/mL, taking the lentivirus out from the -80°C ultra-low temperature refrigerator, and quickly thawing them in a 37°C water bath. Adding polybrene(Sigma, Cat. No.: 107689-10G) to the prepared T cells to a final concentration of 6 $\mu$g/mL, adding an appropriate volume of virus(MOI = 25), gently pipetting and mixing it, sealing the culture dish with a parafilm, and centrifuging at 800g for 1 hour at room temperature. After the centrifugation, continuing to culture for 24 hours, changing the medium, and then continuing to culture in a 37°C, 5% $CO_2$ incubator.

17

Massive expansion of T cells

Gently pipetting the Dynabeads®/cell clusters in the culture system every day until they are completely separated and then counting. When the cell density is greater than $1 \times 10^6$ cells/mL, adding X-Vivo 15 medium(containing 200IU/mL IL-2, 10ng/mL IL-7, 5ng/mL IL-15) to adjust the cell density to $0.5 \times 10^5$ cells/mL, and continuing to culture. After expansion and culture to the 10th day, collecting and storing the cells in a cell cryopreservation solution.

CAR-T Positive Rate Detection

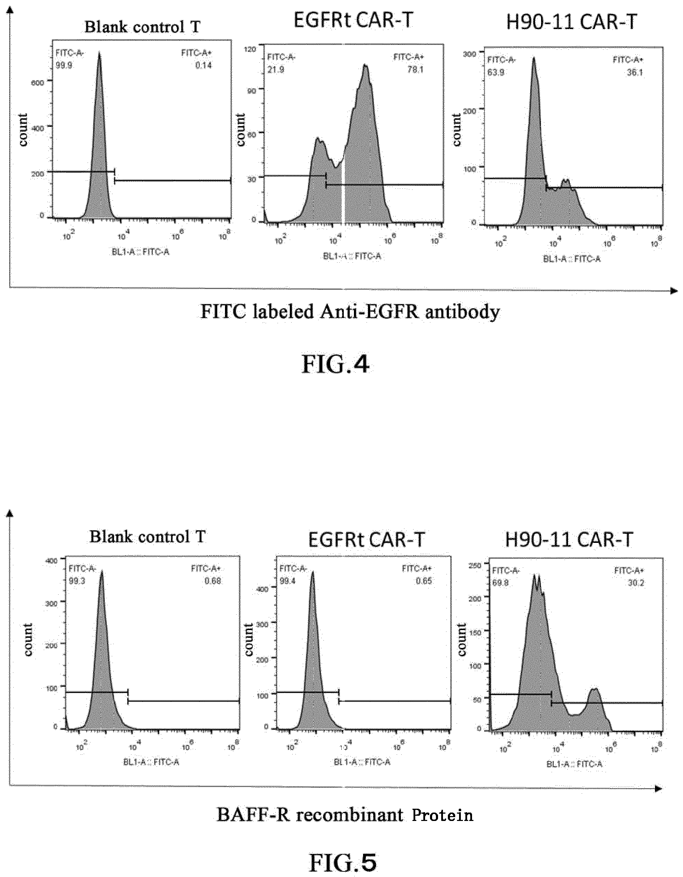

By using FITC-Anti-EGFR antibody(iCARTAB, Cat. No.: IAB006A)(final concentration 10 $\mu$g/mL) to incubate with H90-11 CAR-T(T cells infected with pCDH-EFIa-H90- 11CAR-4-1BB-EGFRt lentivirus ) and EGFRt CAR-T cells(T cells infected with pCDH-EF1a-EGFRt-AT-Free(delete T2A)lentivirus)for 30 minutes at room temperature in the dark, and detecting the expression of EGFRt with flow cytometry. The result is shown in FIG. 4 that the positive rate of the H90-11 CAR-T cells is 36.1%, and the positive rate of the EGFRt CAR-T cells is 78.1%. At the same time, incubating the H90-11 CAR-T and EGFRt CAR-T cells with BAFF-R Liama IgG2b Fc Tag recombinant protein(Acro, Cat. No.: BAR-H5258)(final concentration 10 $\mu$g/mL)respectively. After incubation at room temperature for 30 minutes and centrifugation at 500 g for 5 minutes, discarding the supernatant and diluting FITC-anti Liama IgG(H+L)(Novus, Cat. No.: NBP1-47627) with 0.5% BSA PBS at a ratio of 1:1000. Resuspending the cell pellet at 100 $\mu$L/sample, culturing in the dark at room temperature for 30 minutes, and detecting the positive rate of CAR-T cells with FACS. The result is shown in FIG. 5 that he positive rate of H90-11 CAR-T cells is 30.2%, and the positive rate of EGFRt CAR-T cells is 0.65%. H90-11 CAR-T cells express both EGFRt protein and anti-BAFF-R CAR molecules, and the positive rate is basically the same; while EGFRt CAR-T cells only express EGFRt protein but not anti-BAFF-R CAR molecules, indicating that both H90-11 CAR-T and EGFRt CAR-T cells were successfully prepared.

**Example 5 Evaluation of Tumor Cell killing of Chimeric Antigen Receptor T Cells Targeting BAFF-R *in Vitro***

Lysis of target cells by CAR-T cells

Nalm6-luciferase cells are acute lymphoblastic leukemia cells highly expressing human BAFF-R, which are cells overexpressing Luciferase(luciferase) obtained by lentiviral infection of Nalm6 cells(purchased from Genio Biotech). The cytotoxicity experiment of H90-11 CAR-T cells to Nalm6-luciferase target cells was detected with Luciferase detection kit(Promega, Cat. No.: E2610), and EGFRt CAR-T cells were used as a control. The specific steps are as follows: Resuspending Nalm6-luciferase target cells in complete medium(RPMI1640+10% FBS), inoculating target cells into 96-well plates at $2 \times 10^4$ cells/well, placing them in a 37°C, 5% CO2 incubator, and culturing overnight. Collecting the prepared CAR-T cells by centrifugation and resuspending them in 1640 medium with 10% FBS. Taking the 96-well plates out of the incubator, removing the medium in the wells, and washing the cells gently once with sterile PBS. Adding CAR-T cells with an effect-to-target ratio of 0.5:1, 1:1, 2.5:1, 5:1, and 10:1, setting up duplicate wells and making the final volume to 200 $\mu$L/well. Inoculating the Maxi lysis wells and Mini lysis wells with a same number of target cells, but without CAR-T cells. Placing the 96-well plate in a 37°C, 5% CO2 incubator and incubating it for 18 hours. After the culture was completed, according to the instructions of the Luciferase Detection Kit(Promega, Cat. No.: E2610), detecting the luciferase activity which reflects the ability of the recombinant CAR-T cells in lysing the target cells.

Calculation formula for target cell lysis percentage:

$$\text{Lysis\%} = (1 - [\text{RLU}]\_\text{Sample}/[\text{RLU}]\_\text{Max}) \times 100\%$$

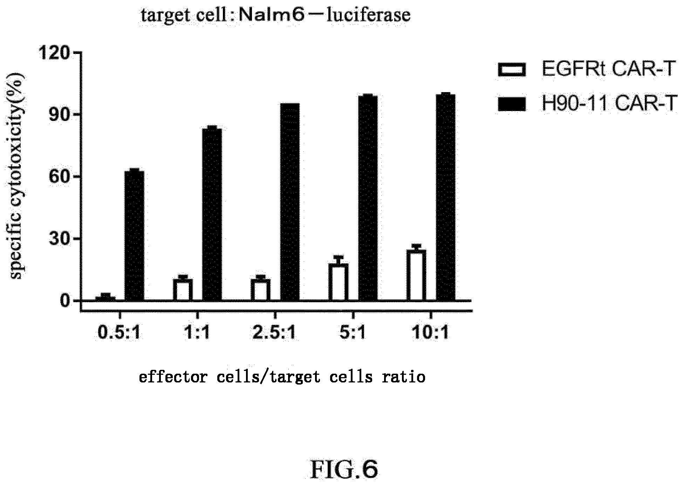

The results are shown in FIG. 6. The killing effect of H90-11 CAR-T cells on Nalm6-luciferase cells expressing BAFF-R is much higher than that of EGFRt CAR-T group, and when the effective target ratios are 0.5:1, 1: 1. At 2.5:1, 5:1, and 10:1, the killing rates of H90-11 CAR-T on target cells are 62.7%, 83.1%, 95.4%, 98.9, and 99.7%, respectively, and the killing rates of the EGFRt CAR-T group on target cells are 2.1%, 10.5%, 10.5%, 18.1, 24.7%, respectively. It indicates that the chimeric antigen receptor T cell targeting BAFF-R prepared by the method of the present invention has strong tumor killing ability.

Detection of CAR-T cytokine secretion level

The expression levels of IL-2 and IFN-$\gamma$ in the co-culture supernatant of Nalm6-luciferase of target cells and prepared CAR-T cells were detected by ELISA method, and the specific experimental steps were as follows: Resuspending Nalm6-luciferase target cells in complete medium, seeding them into 96-well plates at $2 \times 10^4$/well, and

culturing them overnight in a 37°C, 5% CO2 incubator. Using recombinant H90-11 CAR-T cells and EGFRt CAR-T cells as effector cells respectively to establish co-culture systems of effector cells and target cells, and the effect-to-target ratios were 0.5:1, 1:1, 2.5:1, 5:1 and 10:1, setting up duplicated holes. Placing the plate in a 37°C, 5% CO2 incubator and incubating for 18 hours. After the incubation, taking the plate out of the incubator, centrifuging the 96-well plate at room temperature at 1200g for 5 minutes, gently taking out the plate, transferring the supernatant and detecting the expression level of IL-2 and IFN-γ in the supernatant with IL-2(Fomex Bio, Cat. No.: FMS-ELH003) and IFN-γ(Fomex Bio, Cat. No.: FMS-ELH035)detection kits(see the ELISA detection kit manual for specific steps) .

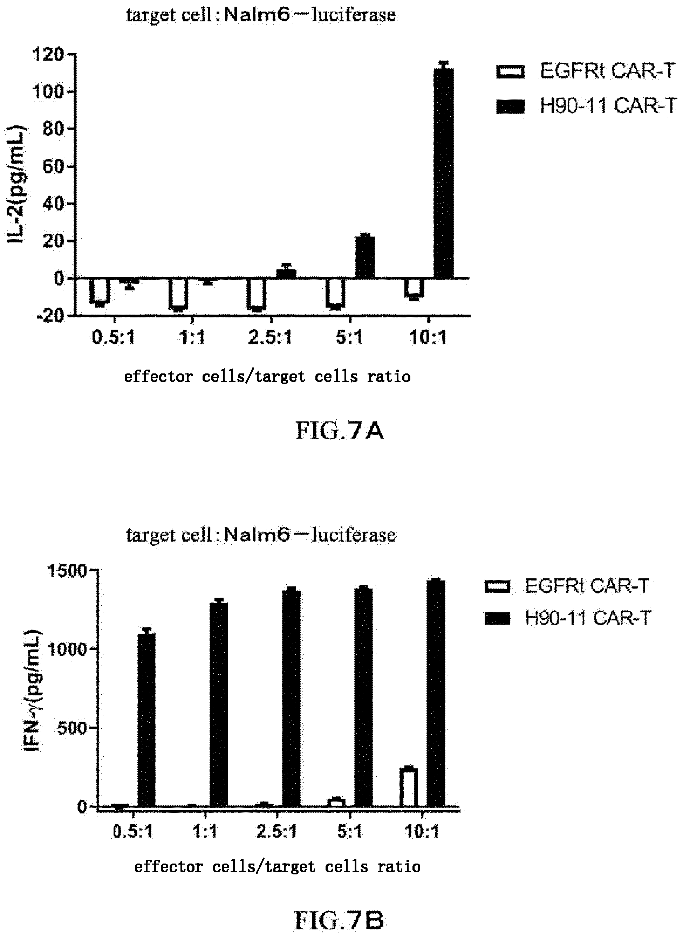

Results are shown in FIG.7A(IL-2) and 7B(IFN-γ), in the supernatant of Nalm6-luciferase cells expressing BAFF-R co-cultured with H90-11 CAR-T, the cytokine levels of IL-2 and IFN-γ cell are significantly higher than that in the EGFRt CAR-T group, and are increased in a gradient manner. The results show that H90-11 CAR-T cells can secrete Th1 type cytokines under the stimulation of target cells expressing BAFF-R.

**Example 6 Evaluation Tumor Cell Killing of Chimeric Antigen Receptor T Cells Targeting BAFF-R *in vivo* in Mice**

The acute lymphoblastic leukemia Nalm6-luciferase cell line was xenografted into an NSG mouse model to evaluate the *in vivo* activity of H90-11 CAR-T.

20 NSG mice aged 6-8 weeks(purchased from Beijing Biocytogen Biotechnology Co., Ltd.)(body weight 18-22g) were taken, and after one week of adaptation, Nalm6-luciferase was inoculated via the tail veins, and each mouse was inoculated with $1\times10^6$ tumor cells. 5 days after inoculation of tumor cells in the tail vein, the first *in vivo* imaging was performed, and the imaging effect was not satisfactory. 8 days after inoculation, mice with unsuccessful modeling were excluded from subsequent experimental groups. Mice were grouped according to the *in vivo* imaging results, and the mice were randomly divided into two groups, with 8 mice in each group, and $2.25\times10^6$ H90-11 CAR-T cells or EGFRt CAR-T cells were infused through the tail vein, and the mice were injected intraperitoneally with D-luciferin(Shanghai Lechen, Cat. No.: BC219-10) every 7 days and the mice were anesthetized using a gas anesthesia machine at the same time. Tumor live imaging data were collected, and the body weight changes of the mice were recorded and the survival curve of the mice was drawn.

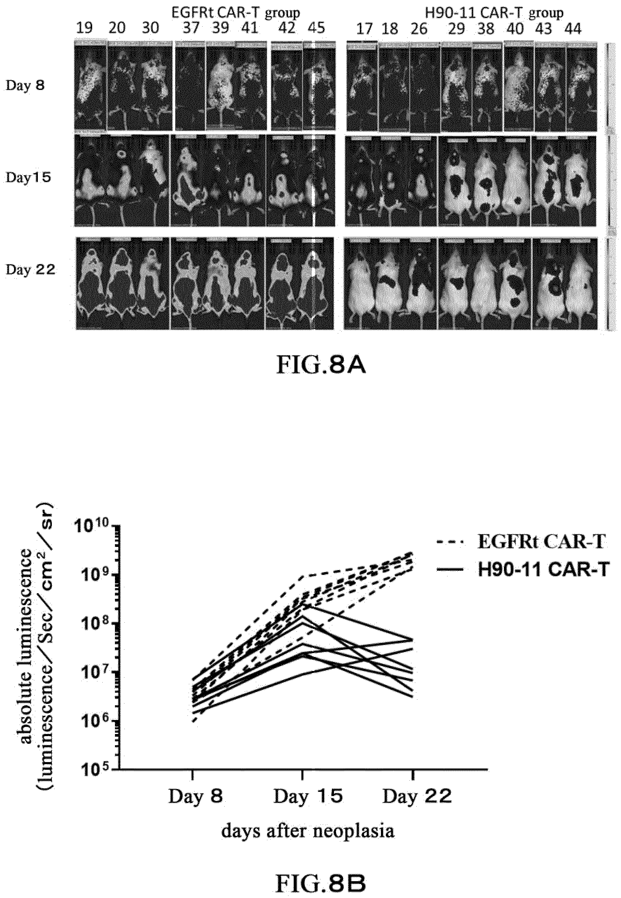

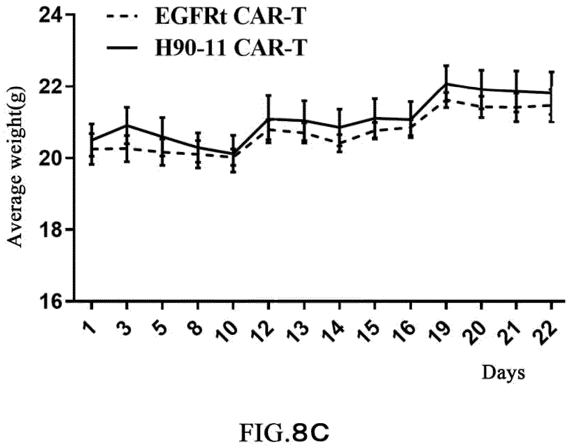

On the 8th, 15th, and 22nd days after the modeling, the small animal in vivo imaging equipment was used to conduct *in vivo* imaging observations. The imaging results are shown in FIG. 8A, and the absolute luminescence curve drawn is shown in FIG. 8B. According to the imaging results, compared with the EGFRt CAR-T control group, H90-11 CAR-T had a significant killing effect on tumor cells; after CAR-T cell infusion on the 8th day, compared with the EGFRt CAR-T infusion control group, the increase in tumor burden(absolute luminescence) of mice in the H90-11 CAR-T infusion group slowed down from the infusion to the 15th day, and the tumor burden decreased rapidly after the 15th day. The drawn body weight change curve of the mice is shown in FIG. 8C. The body weight of mice infused with EGFRt CAR-T control cells was comparable to that of mice infused with H90-11 CAR-T cells; the slowly increase of mice body weight was maintained during the test, indicating that CAR-T cell infusion do not cause severe toxic reactions.

**Claims**

1. A chimeric antigen receptor molecule specifically recognizing BAFF-R, comprising a specific recognition domain and an activation stimulation domain, and a transmembrane domain located between the specific recognition domain and the activation stimulation domain, wherein, the specific recognition domain comprises a heavy chain variable region and a light chain variable region, wherein,

   the heavy chain variable region comprises three complementary determining regions CDR H1, CDR H2 and CDR H3, and CDR H1 comprises an amino acid sequence set forth in SEQ ID NO:6, CDR H2 comprises an amino acid sequence set forth in SEQ ID NO:7, and CDR H3 comprises an amino acid sequence set forth in SEQ ID NO: 8; the light chain variable region comprises CDR L1, CDR L2 and CDR L3, and the CDR L1 comprises an amino acid sequence set forth in SEQ ID NO:10, CDR L2 comprises an amino acid sequence set forth in SEQ ID NO: 11, and CDR L3 comprises an amino acid sequence set forth in SEQ ID NO:12.

2. The chimeric antigen receptor molecule specifically recognizing BAFF-R according to claim 1, wherein the heavy chain variable region and the light chain variable region further comprise a human antibody framework region.

3. The chimeric antigen receptor molecule specifically recognizing BAFF-R according to claim 1 or 2, wherein the heavy chain variable region comprises an amino acid sequence set forth in SEQ ID NO: 5 or an amino acid sequence having from 80% to 100% sequence identity therewith; and the light chain variable region comprises an amino acid sequence set forth in SEQ ID NO: 9 or an amino acid sequence having from 80% to 100% sequence identity therewith.

4. The chimeric antigen receptor molecule specifically recognizing BAFF-R according to any one of claims 1 to 3, wherein the activation stimulation domain comprises a signaling domain, and the signaling domain comprises an immune receptor tyrosine activation motif.

5. The chimeric antigen receptor molecule specifically recognizing BAFF-R according to claim 4, wherein the activation stimulation domain further comprises one, two, three or more costimulatory domains.

6. The chimeric antigen receptor molecule specifically recognizing BAFF-R according to claim 5, wherein the costimulatory domain comprises the amino acid sequence of the human 4-1BB molecular(4-1BB) intracellular region set forth in SEQ ID NO: 18 or an amino acid sequence having from 80% to 100% sequence identity therewith.

7. The chimeric antigen receptor molecule specifically recognizing BAFF-R according to any one of claims 1 to 6, wherein the transmembrane domain comprises a transmembrane domain selected from the group consisting of: transmembrane domain of CD4, CD8α, CD28 or CD3ζ.

8. The chimeric antigen receptor molecule specifically recognizing BAFF-R according to any one of claims 1 to 7, wherein the transmembrane domain and the specific recognition domain are linked by a hinge region, and the hinge region is selected from the group consisting of the hinge regions of IgG, IgD, or CD8α/CD28.

9. A nucleic acid molecule encoding a chimeric antigen receptor molecule specifically recognizing BAFF-R according to any one of claims 1 to 8.

10. The nucleic acid molecule according to claim 9, comprising a polynucleotide sequence set forth in SEQ ID NO: 3 or a polynucleotide sequence having from 80% to 100% sequence identity therewith.

11. The nucleic acid molecule according to claim 9 or 10, further comprising a coding sequence of a membrane localization signal peptide molecule.

12. The nucleic acid molecule according to claim 11, which from the 5' end to the 3' end comprises the following polynucleotide sequences or polynucleotide sequences having from 80% to 100% sequence identity therewith: SEQ ID NO: 1. SEQ ID NO:3, SEQ ID NO: 13, SEQ ID NO:15, SEQ ID NO:17 and SEQ ID NO:19.

13. An engineered immune effector cell comprising the chimeric antigen receptor molecule specifically recognizing BAFF-R according to any one of claims 1 to 8 or the nucleic acid molecule according to any one of claims 9 to 12.

14. A pharmaceutical composition comprising a therapeutically effective amount of the chimeric antigen receptor molecule specifically recognizing BAFF-R according to any one of claims 1 to 8, the nucleic acid molecule according to any one of claims 9 to 12, or the engineered immune effector cell according to claim 13.

15. The pharmaceutical composition of claim 14 for use in treating or preventing a disease, wherein the disease is selected from mantle cell lymphoma, follicular lymphoma, diffuse large B-cell lymphoma, marginal zone lymphoma, Burkitt's Lymphoma, lymphoblastic leukemia, chronic lymphocytic leukemia, hairy cell leukemia, rheumatoid arthritis, systemic lupus erythematosus, multiple sclerosis, glomerular nephritis, Sjögren's syndrome, or autoimmune hemolytic anemia.

**Patentansprüche**

1. Chimäres Antigenrezeptormolekül, das spezifisch BAFF-R erkennt, umfassend eine spezifische Erkennungsdomäne und eine Aktivierungs-Stimulierungs-Domäne sowie eine Transmembrandomäne, die sich zwischen der spezifischen Erkennungsdomäne und der Aktivierungs-Stimulierungs-Domäne befindet, wobei die spezifische Erkennungsdomäne eine variable Region der schweren Kette und eine variable Region der leichten Kette umfasst, wobei

die variable Region der schweren Kette drei komplementaritätsbestimmende Regionen CDR H1, CDR H2 und CDR H3 umfasst und die CDR H1 eine wie in SEQ ID NO:6 angegebene Aminosäuresequenz umfasst, die CDR

EP 4 342 913 B1

H2 eine wie in SEQ ID NO:7 angegebene Aminosäuresequenz umfasst und die CDR H3 eine wie in SEQ ID NO: 8 angegebene Aminosäuresequenz umfasst,
die variable Region der leichten Kette CDR L1, CDR L2 und CDR L3 umfasst und die CDR L1 eine wie in SEQ ID NO:10 angegebene Aminosäuresequenz umfasst, die CDR L2 eine wie in SEQ ID NO: 11 angegebene Aminosäuresequenz umfasst und die CDR L3 eine wie in SEQ ID NO:12 angegebene Aminosäuresequenz umfasst.

2. Chimäres Antigenrezeptormolekül, das spezifisch BAFF-R erkennt, gemäß Anspruch 1, wobei die variable Region der schweren Kette und die variable Region der leichten Kette ferner eine menschliche Antikörper-Framework-Region umfassen.

3. Chimäres Antigenrezeptormolekül, das spezifisch BAFF-R erkennt, gemäß Anspruch 1 oder 2, wobei die variable Region der schweren Kette eine wie in SEQ ID NO: 5 angegebene Aminosäuresequenz oder eine Aminosäuresequenz mit 80%iger bis 100%iger Sequenzidentität damit umfasst und die variable Region der leichten Kette eine wie in SEQ ID NO: 9 angegebene Aminosäuresequenz oder eine Aminosäuresequenz mit 80%iger bis 100%iger Sequenzidentität damit umfasst.

4. Chimäres Antigenrezeptormolekül, das spezifisch BAFF-R erkennt, gemäß einem der Ansprüche 1 bis 3, wobei die Aktivierungs-Stimulierungs-Domäne eine Signaldomäne umfasst und die Signaldomäne ein Immunrezeptor-Tyrosin-basiertes Aktivierungsmotiv umfasst.

5. Chimäres Antigenrezeptormolekül, das spezifisch BAFF-R erkennt, gemäß Anspruch 4, wobei die Aktivierungs-Stimulierungs-Domäne ferner eine, zwei, drei oder mehr kostimulatorische Domänen umfasst.

6. Chimäres Antigenrezeptormolekül, das spezifisch BAFF-R erkennt, gemäß Anspruch 5, wobei die kostimulatorische Domäne die in SEQ ID NO: 18 angegebene Aminosäuresequenz der menschlichen 4-1BB-molekularen(4-1BB) intrazellulären Region oder eine Aminosäuresequenz mit 80%iger bis 100%iger Sequenzidentität damit umfasst.

7. Chimäres Antigenrezeptormolekül, das spezifisch BAFF-R erkennt, gemäß einem der Ansprüche 1 bis 6, wobei die Transmembrandomäne eine Transmembrandomäne umfasst, die ausgewählt ist aus der Gruppe bestehend aus den Transmembrandomänen CD4, CD8α, CD28 und CD3ζ.

8. Chimäres Antigenrezeptormolekül, das spezifisch BAFF-R erkennt, gemäß einem der Ansprüche 1 bis 7, wobei die Transmembrandomäne und die spezifische Erkennungsdomäne durch eine Gelenkregion verbunden sind und die Gelenkregion ausgewählt ist aus der Gruppe bestehend aus den Gelenkregionen IgG, IgD und CD8α/CD28.

9. Nukleinsäuremolekül, das ein chimäres Antigenrezeptormolekül, das spezifisch BAFF-R erkennt, gemäß einem der Ansprüche 1 bis 8 kodiert.

10. Nukleinsäuremolekül gemäß Anspruch 9, umfassend eine wie in SEQ ID NO: 3 angegebene Polynukleotidsequenz oder eine Polynukleotidsequenz mit 80%iger bis 100%iger Sequenzidentität damit.

11. Nukleinsäuremolekül gemäß Anspruch 9 oder 10, ferner umfassend eine Kodierungssequenz eines Membranlokalisierungssignalpeptidmoleküls.

12. Nukleinsäuremolekül gemäß Anspruch 11, das vom 5'-Ende zum 3'-Ende die folgenden Polynukleotidsequenzen oder Polynukleotidsequenzen mit 80%iger bis 100%iger Sequenzidentität damit umfasst: SEQ ID NO: 1, SEQ ID NO:3, SEQ ID NO:13, SEQ ID NO:15, SEQ ID NO:17 und SEQ ID NO:19.

13. Veränderte Immuneffektorzelle, die das chimäre Antigenrezeptormolekül, das spezifisch BAFF-R erkennt, gemäß einem der Ansprüche 1 bis 8 oder das Nukleinsäuremolekül gemäß einem der Ansprüche 9 bis 12 umfasst.

14. Pharmazeutische Zusammensetzung, die eine therapeutisch wirksame Menge des chimären Antigenrezeptormoleküls, das spezifisch BAFF-R erkennt, gemäß einem der Ansprüche 1 bis 8, des Nukleinsäuremoleküls gemäß einem der Ansprüche 9 bis 12 oder der veränderten Immuneffektorzelle gemäß Anspruch 13 umfasst.

15. Pharmazeutische Zusammensetzung nach Anspruch 14 zur Verwendung bei der Behandlung oder Prävention einer Erkrankung, wobei die Erkrankung ausgewählt ist aus Mantelzell-Lymphom, follikulärem Lymphom, diffusem großzelligem B-Zell-Lymphom, Marginalzonenlymphom, Burkitt-Lymphom, lymphoblastischer Leukämie, chronischer lymphatischer Leukämie, Haarzell-Leukämie, rheumatoider Arthritis, systemischem Lupus erythematodes, multipler Sklerose, Glomerulonephritis, Sjögren-Syndrom oder autoimmunhämolytischer Anämie.

## Revendications

1. Molécule de récepteur antigénique chimère reconnaissant spécifiquement BAFF-R, comprenant un domaine de reconnaissance spécifique et un domaine de stimulation d'activation, et un domaine transmembranaire situé entre le domaine de reconnaissance spécifique et le domaine de stimulation d'activation, dans laquelle le domaine de reconnaissance spécifique comprend une région variable de chaîne lourde et une région variable de chaîne légère, dans laquelle

   la région variable de chaîne lourde comprend trois régions de détermination de complémentarité CDR H1, CDR H2 et CDR H3, et la CDR H1 comprend une séquence d'acides aminés définie dans la SEQ ID NO: 6, la CDR H2 comprend une séquence d'acides aminés définie dans la SEQ ID NO : 7 et la CDR H3 comprend une séquence d'acides aminés définie dans la SEQ ID NO : 8 ;
   la région variable de chaîne légère comprend les CDR L1, CDR L2 et CDR L3, et la CDR L1 comprend une séquence d'acides aminés définie dans la SEQ ID NO : 10, la CDR L2 comprend une séquence d'acides aminés définie dans la SEQ ID NO : 11 et la CDR L3 comprend une séquence d'acides aminés définie dans la SEQ ID NO : 12.

2. Molécule de récepteur antigénique chimère reconnaissant spécifiquement BAFF-R selon la revendication 1, dans laquelle la région variable de chaîne lourde et la région variable de chaîne légère comprennent en outre une région charpente d'anticorps humain.

3. Molécule de récepteur antigénique chimère reconnaissant spécifiquement BAFF-R selon la revendication 1 ou 2, dans laquelle la région variable de chaîne lourde comprend une séquence d'acides aminés définie dans la SEQ ID NO : 5 ou une séquence d'acides aminés ayant une identité de séquence de 80 % à 100 % avec celle-ci ; et la région variable de chaîne légère comprend une séquence d'acides aminés définie dans la SEQ ID NO : 9 ou une séquence d'acides aminés ayant une identité de séquence de 80 % à 100 % avec celle-ci.

4. Molécule de récepteur antigénique chimère reconnaissant spécifiquement BAFF-R selon l'une quelconque des revendications 1 à 3, dans laquelle le domaine de stimulation d'activation comprend un domaine de signalisation, et le domaine de signalisation comprend un motif d'activation basé sur la tyrosine des immunorécepteurs.

5. Molécule de récepteur antigénique chimère reconnaissant spécifiquement BAFF-R selon la revendication 4, dans laquelle le domaine de stimulation d'activation comprend en outre un, deux, trois ou plus de trois domaines co-stimulateurs.

6. Molécule de récepteur antigénique chimère reconnaissant spécifiquement BAFF-R selon la revendication 5, dans laquelle le domaine co-stimulateur comprend la séquence d'acides aminés de la région intracellulaire de la molécule humaine 4-1BB (4-1BB) indiquée dans la SEQ ID NO : 18 ou une séquence d'acides aminés ayant une identité de séquence de 80 % à 100 % avec celle-ci.

7. Molécule réceptrice d'antigène chimère reconnaissant spécifiquement BAFF-R selon l'une quelconque des revendications 1 à 6, dans laquelle le domaine transmembranaire comprend un domaine transmembranaire choisi dans le groupe consistant en domaine transmembranaire de CD4, CD8$\alpha$, CD28 ou CD3$\zeta$.

8. Molécule de récepteur antigénique chimère reconnaissant spécifiquement BAFF-R selon l'une quelconque des revendications 1 à 7, dans laquelle le domaine transmembranaire et le domaine de reconnaissance spécifique sont liés par une région charnière, et la région charnière est choisie dans le groupe consistant en les régions charnières de IgG, IgD ou CD8$\alpha$/CD28.

9. Molécule d'acide nucléique codant pour une molécule de récepteur antigénique chimère reconnaissant spécifiquement BAFF-R selon l'une quelconque des revendications 1 à 8.

10. Molécule d'acide nucléique selon la revendication 9, comprenant une séquence polynucléotidique définie dans la SEQ ID NO : 3 ou une séquence polynucléotidique ayant une identité de séquence de 80 % à 100 % avec celle-ci.

11. Molécule d'acide nucléique selon la revendication 9 ou 10, comprenant en outre une séquence codante d'une molécule de peptide signal de localisation membranaire.

12. Molécule d'acide nucléique selon la revendication 11, qui comprend, de l'extrémité 5' à l'extrémité 3', les séquences polynucléotidiques suivantes ou des séquences polynucléotidiques ayant une identité de séquence de 80 % à 100 % avec celles-ci : SEQ ID NO : 1, SEQ ID NO : 3, SEQ ID NO : 13, SEQ ID NO : 15, SEQ ID NO 17 et SEQ ID NO : 19.

13. Cellule effectrice immunitaire modifiée par génie génétique comprenant la molécule de récepteur antigénique chimère reconnaissant spécifiquement BAFF-R selon l'une quelconque des revendications 1 à 8 ou la molécule d'acide nucléique selon l'une quelconque des revendications 9 à 12.

14. Composition pharmaceutique comprenant une quantité thérapeutiquement efficace de la molécule de récepteur antigénique chimère reconnaissant spécifiquement BAFF-R selon l'une quelconque des revendications 1 8, de la molécule d'acide nucléique selon l'une quelconque des revendications 9 à 12, ou de la cellule effectrice immunitaire modifiée par génie génétique selon la revendication 13.

15. Composition pharmaceutique selon la revendication 14 pour une utilisation dans le traitement ou la prévention d'une maladie, la maladie étant choisie parmi le lymphome à cellules du manteau, le lymphome folliculaire, le lymphome diffus à grandes cellules B, le lymphome de la zone marginale, le lymphome de Burkitt, la leucémie lymphoblastique, la leucémie lymphoïde chronique, la leucémie à tricholeucocytes, la polyarthrite rhumatoïde, le lupus érythémateux disséminé, la sclérose en plaques, la néphrite glomérulaire, le syndrome de Sjögren ou l'anémie hémolytique auto-immune.

H90-11 CAR

| EF1α | CD8a Signal peptide | H90-11 scFV | CD8 hinge region | CD8 TM | 4-1BB | CD3Zeta |

FIG.1

FIG.2

FIG.3

FITC labeled Anti-EGFR antibody

FIG.4

BAFF-R recombinant Protein

FIG.5

target cell: Nalm6 — luciferase

FIG.6

target cell：Nalm6－luciferase

FIG.7A

target cell：Nalm6－luciferase

FIG.7B

FIG.8A

FIG.8B

FIG.8C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017214167 A1
- EP 404097 A
- WO 9311161 A
- US 5641870 A
- US 20210261676 A

### Non-patent literature cited in the description

- **KABAT et al.** Sequences of Proteins of Immunological Interest. NIH Publication, 1991, vol. 1-3, 91-3242
- **WARD et al.** *Nature*, 1989, vol. 341, 544-546
- **BIRD et al.** *Science*, 1988, vol. 242, 423-426
- **HUSTON et al.** *PNAS(USA)*, 1988, vol. 85, 5879-5883
- **HOLLINGER et al.** *Proc. Natl .Acad.Sci.USA*, 1993, vol. 90, 6444-6448
- **ZAPATA et al.** *Protein Eng*, 1995, vol. 8 (10), 1057-1062
- Remington: The Science and Practice of Pharmacy. Pharmaceutical Press, 2012